(19) Europäisches Patentamt
European Patent Office
Office européen des brevets

(11) Publication number: **0 470 856 B1**

(12) **EUROPEAN PATENT SPECIFICATION**

(45) Date of publication of patent specification: **11.10.95**

(51) Int. Cl.[6]: **C07D 261/08**, A01N 43/80, C07D 261/10, C07D 261/18, C07D 261/14

(21) Application number: **91307351.6**

(22) Date of filing: **09.08.91**

(54) **Isoxazole derivatives, process for their preparation and their herbicidal applications.**

(30) Priority: **10.08.90 GB 9017539**

(43) Date of publication of application:
**12.02.92 Bulletin 92/07**

(45) Publication of the grant of the patent:
**11.10.95 Bulletin 95/41**

(84) Designated Contracting States:
**AT BE CH DE DK ES FR GB GR IT LI LU NL SE**

(56) References cited:
**EP-A- 0 002 480**
**EP-A- 0 248 399**
**GB-A- 1 274 578**
**GB-A- 1 304 558**
**GB-A- 2 018 247**
**EP-A- 0 241 232**
**GB-A- 1 268 745**
**GB-A- 1 298 535**
**GB-A- 1 305 863**
**US-A- 4 562 187**

(73) Proprietor: **RHONE-POULENC AGRICULTURE LTD.**
**Fyfield Road**
**Ongar,**
**Essex CM5 0HW (GB)**

(72) Inventor: **Cain, Paul A., Res. Station of Rhone-Poulenc**
**Agriculture Ltd.,**
**Fyfield Road**
**Ongar,**
**Essex CM5 0HW (GB)**
Inventor: **Cramp, Susan Mary, Res. Station of Rhone-Poulenc**
**Agriculture Ltd.,**
**Fyfield Road**
**Ongar,**
**Essex CM5 0HW (GB)**
Inventor: **Little, Gillian M., Res. Station of Rhone-Poulenc**
**Agriculture Ltd.,**
**Fyfield Road**
**Ongar,**
**Essex CM5 0HW (GB)**

(74) Representative: **Bentham, Stephen et al**
**J.A. KEMP & CO.**
**14 South Square**
**Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 470 856 B1

## Description

This invention relates to novel 4-benzyl isoxazole derivatives, compositions containing them and their use as herbicides. The present invention relates to 4-benzyl isoxazole derivatives of general formula (I):

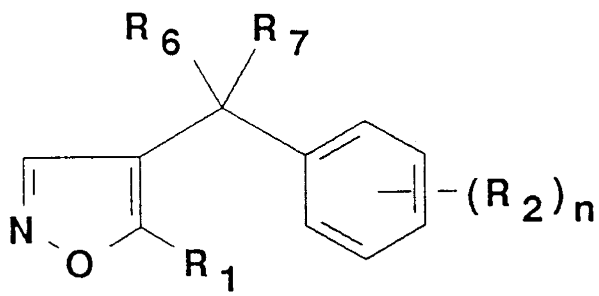

(I)

wherein $R_1$ represents:

a straight- or branched- chain alkyl, alkenyl or alkynyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$; or

a cycloalkenyl group containing 5 or 6 carbon atoms which may be optionally substituted by one or more $R_5$ groups or one or more halogen atoms or a group $COOR_5$; or

an aryl or aralkyl (e.g. benzyl) group of general formula $[(R_2)_q$ phenyl$]-[C(R_3)(R_4)]_p-$ (aryl is generally C6-C10 and aralkyl is generally C7-C11); or

an ester group, $COOR_5$; or

an aldehyde or acyl group, $COR_3$; or

a cyano or nitro group; or

an amino group, $-NR_3R_4$; or

a halogen atom (i.e. F,Cl,Br,I);

$R_2$ represents:

a nitro or cyano group; or

a halogen atom (i.e. F,Cl,Br,I); or

a group, $R_5$; or

a sulphenyl, sulphinyl or sulphonyl group, $-S(O)_mR_5$; or

a sulphamoyl group, $-SO_2NR_3R_4$; or

an ester group, $-COOR_5$; or

an acyl or aldehyde group, $-COR_3$; or

a carbamoyl or thiocarbamoyl group $-CONR_3R_4$ or $-CSNR_3R_4$; or

an alkoxy group, $-OR_5$; or

an alkyl group (with 1 to 3 carbon atoms) substituted by $-OR_5$;

$R_3$ and $R_4$, which may be the same or different, each represents:

a hydrogen atom or a straight- or branched- chain alkyl group containing up to 6 carbon atoms which is optionally substituted by one or more halogen atoms;

$R_5$ represents:

a straight- or branched-chain alkyl group containing up to 6 carbon atoms which is optionally substituted by one or more halogen atoms;

$R_6$ represents:

a hydrogen atom; or

a hydroxy group, OH; or

a halogen atom (i.e. F,Cl,Br,I); or

a group $R_5$; or

an alkenyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$; or

a sulphenyl, sulphinyl or sulphonyl group, $-S(O)_mR_5$; or
an ester group, $-COOR_5$; or
a cyano group; or
an acyl or aldehyde group, $-COR_3$; or
a carbamoyl or thiocarbamoyl group, $-CONR_3R_4$ or $-CSNR_3R_4$;

or

an alkoxy group, $-OR_5$; or
a phenoxy group, -O-phenyl-$(R_2)_q$; or
a benzoyloxy group, $-OCH_2$-phenyl-$(R_2)$q; or
an acyloxy group, $-OCOR_8$; or
a benzoyloxy group, -OCO-phenyl-$(R_2)$q; or
a group -OCO-Het1; or
a carbamoyloxy group, $-OCONR_3R_4$; or
an alkylsulphonyloxy group, $-OSO_2R_8$; or
a phenylsulphonyloxy group, $-OSO_2$-phenyl-$(R_2)$q
a sulphamoyloxy group, $-OSO_2NR_3R_4$; or
an amino group, $-NR_3R_4$; or
an acylamino group, $-NR_3COR_5$; or
a group Het2;

$R_7$ represents:
a hydrogen atom; or
a group, $R_5$;

or $R_6$ and $R_7$ may form with the carbon atom to which they are attached, a ketal $((OR_5)_2)$, or a thioketal (-$(SR_5)_2$), or a cyclic ketal or cyclic thioketal containing 5 or 6 atoms in the ring optionally substituted by one or more $R_5$ groups;

$R_8$ represents:

a straight- or branched- chain alkyl or alkenyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$;

Het1 represents a heterocycle containing 5 or 6 atoms in the ring, one or more of which is a heteroatom selected from nitrogen, sulphur and oxygen, the ring carbon atoms being optionally substituted by a group $R_2$;

Het2 represents a heterocyclic group selected from: pyrrol-l-yl, pyrazol-l-yl, imidazol-l-yl, 1,2,4-triazol-4-yl, 1,2,4-triazoyl-l-yl, 1,2,3-triazol-l-yl or 1,2,3-triazol-2-yl; each of which may be optionally substituted by one or more groups $R_2$;

m represents zero, 1, or 2;
n represents an integer from 1 to 5;
q represents zero or an integer from 1 to 5;
p represents zero or 1;
and agriculturally acceptable salts thereof, which possess valuable herbicidal properties.

It is to be understood that when n or q is an integer from 2 to 5 the substituents represented by $R_2$ may be the same or different.

Furthermore in certain cases the substituents $R_1$, $R_2$, $R_3$, $R_4$, $R_5$, $R_6$, $R_7$ and $R_8$ contribute to optical and/or stereoisomerism. All such forms are embraced by the present invention.

By the term "agriculturally acceptable salts" is meant salts the cations or anions of which are known and accepted in the art for the formation of salts for agricultural or horticultural uses. Preferably the salts are water-soluble. Suitable salts with bases include alkali metal (e.g. sodium and potassium), ammonium and amine (e.g. diethanolamine, triethanolamine, octylamine, morpholine and dioctylmethylamine) salts. Suitable acid addition salts of the compounds of general formula (I), which may incorporate an amino radical, include salts with inorganic acids, for example hydrochlorides, sulphates, phosphates and nitrates and salts with organic acids, for example acetic acid.

It is to be understood that where reference is made in the present specification to the compounds of general formula (I), such reference is intended to include also the salts with agriculturally acceptable acids or bases of compounds of general formula (I) where appropriate.

GB-A-1 274 578 and GB-A-1 304 558 describe N-benzylimidazoles and N-benzyltriazoles useful for the regulation of plant growth.

Preferred compounds of formula (I) are those wherein

a) one of the groups represented by $(R_2)_n$ is in the ortho position on the phenyl ring; and/or

b) where there are two groups represented by $(R_2)$n they are in the 2-and 4- positions on the phenyl ring; and/or

c) $R_1$ is alkyl optionally substituted by halogen; or cycloalkyl optionally substituted by alkyl or halogen; most preferably cyclopropyl, 1-methylcyclopropyl or isopropyl; and/or

d) $R_2$ is halogen; or nitro; or $R_5$; or $-S(O)_mR_5$; or $-OR_5$; or alkyl substituted by $-OR_5$; or $-COOR_5$; and/or

e) $R_5$ is alkyl (with 1 to 3 carbon atoms) optionally substituted by fluorine or chlorine; and/or

f) $R_6$ is hydrogen, halogen, -OH, $-OR_5$, $-OCOR_8$, or alkyl (with one to four carbon atoms) optionally substituted by one or more halogen atoms; most preferably halogen, -OH, $-OR_5$ or $-OCOR_8$; and/or

g) One of the groups $R_6$ or $R_7$ is a hydrogen atom; and/or

h) $R_7$ is hydrogen or an alkyl group containing from one to four carbon atoms; and/or

i) $R_8$ is an alkyl group containing from one to four carbon atoms optionally substituted by one or more halogen atoms; and/or

j) n is 2 or 3.

Among the particularly preferred compounds of formula (I) because of their herbicidal properties are:

a) 4-[hydroxy-(2-nitro-4-trifluoromethylphenyl)methyl]-5-cyclopropylisoxazole;
b) 4-[hydroxy-(2-nitro-4-trifluoromethylphenyl)methyl]-5-methyl isoxazole;
c) 4-[hydroxy-(2-chloro-3-ethoxy-4-methanesulphonylphenyl)methyl]-5-cyclopropylisoxazole;
d) 4-[acetoxy-(2-nitro-4-trifluoromethylphenyl)methyl]-5-cyclopropylisoxazole;
e) 4-[chloro-(2-nitro-4-trifluoromethylphenyl)methyl]-5-cyclopropylisoxazole;
f) 4-[bromo-(2-nitro-4-trifluoromethylphenyl)methyl]-5-cyclopropylisoxazole;
g) 4-[N,N-dimethylamino-(2-nitro-4-trifluoromethylphenyl)methyl]-5-cyclopropylisoxazole;
h) 4-[hydroxy-(2-chloro-4-methanesulphonylphenyl)methyl]-5-cyclopropylisoxazole;
i) 4-[methoxy-(2-nitro-4-trifluoromethylphenyl)methyl]-5-cyclopropylisoxazole;
j) 4-[hydroxy-(2-nitro-4-methylphenyl)methyl]-5-cyclopropylisoxazole;
k) 4-[hydroxy-(2-chloro-3-ethoxy-4-ethanesulphonylphenyl)methyl]-5-methylisoxazole;
l) 4-[hydroxy-(2-chloro-3-ethoxy-4-ethanesulphonylphenyl)methyl]-5-cyclopropylisoxazole;
m) 4-[acetoxy-(2-chloro-4-methanesulphonylphenyl)methyl]-5-cyclopropylisoxazole;
n) 4-[methoxy-(2-chloro-4-methanesulphonylphenyl)methyl]-5-cyclopropylisoxazole;
o) 4-[hydroxy-(2-chloro-4-fluorophenyl)methyl]-5-cyclopropylisoxazole;
p) 4-[hydroxy-(2-trifluoromethyl-4-methanesulphonylphenyl)methyl]-5-cyclopropylisoxazole;
q) 4-[acetoxy-(2-trifluoromethyl-4-methanesulphonylphenyl)methyl]-5-cyclopropylisoxazole;
r) 4-[methoxy-(2-trifluoromethyl-4-methanesulphonylphenyl)methyl]-5-cyclopropylisoxazole;
s) 4-[bromo-(2-trifluoromethyl-4-methanesulphonylphenyl)methyl]-5-cyclopropylisoxazole;
t) 4-[chloro-(2-trifluoromethyl-4-methanesulphonylphenyl)methyl]-5-cyclopropylisoxazole;
u) 4-[trifluoroacetoxy-(2-trifluoromethyl-4-methanesulphonylphenyl) methyl]-5-cyclopropylisoxazole;
v) 4-[hydroxy-(2-methanesulphonylphenyl)methyl]-5-cyclopropylisoxazole;
w) 4-[hydroxy-(2,3-dichloro-4-methanesulphonylphenyl)methyl]-5-(1-methylcyclopropyl)isoxazole;
x) 4-[hydroxy-(2-methanesulphonyl-4-chlorophenyl)methyl]-5-cyclopropylisoxazole;
y) 4-[hydroxy-(2-bromo-4-methanesulphonylphenyl)methyl]-5-cyclopropylisoxazole;
z) 4-[hydroxy-(2-methanesulphonyl-4-bromophenyl)methyl]-5-cyclopropylisoxazole;
aa) 4-[hydroxy-(2-methyl-4-methanesulphonylphenyl)methyl]-5-cyclopropylisoxazole;
ab) 4-[hydroxy-(2-chloro-4-methanethiophenyl)methyl]-5-cyclopropylisoxazole;
ac) 4-[hydroxy-(2-methanesulphonyl-4-trifluoromethylphenyl)methyl]-5-cyclopropylisoxazole;
ad) 4-[hydroxy-(2-chloro-4-methanesulphinylphenyl)methyl]-5-cyclopropylisoxazole;
ae) 4-[hydroxy-(2-trifluoromethyl-4-methanethiophenyl)methyl]-5-cyclopropylisoxazole;
af) 4-[hydroxy-(2-fluoro-4-methanesulphonylphenyl)methyl]-5-cyclopropylisoxazole;
ag) 4-[hydroxy-(2-methanesulphonyl-4-chlorophenyl)methyl]-5-isopropylisoxazole; and
ah) 4-[hydroxy-(2-methanesulphonyl-4-chlorophenyl)methyl]-5-(1-methylcyclopropyl)isoxazole.

The letters a to ah are assigned to the above compounds for identification and reference hereinafter.

The compounds of general formula (I) can be prepared by the application or adaptation of known methods (i.e. methods heretofore used or described in the chemical literature), for example as hereinafter described.

In the following description where symbols appearing in formulae are not specifically defined it is to be understood that they are "as hereinbefore defined" in accordance with the first definition of each symbol in this specification.

It is to be understood that in the descriptions of the following processes, that the sequences may be performed in different orders and that suitable protecting groups may be required to achieve the

compounds sought.

According to a feature of the present invention the compounds of general formula (I), wherein $R_6$ is an OH group and $R_7$ represents the hydrogen atom, may be prepared by the reduction of a compound of general formula (II):

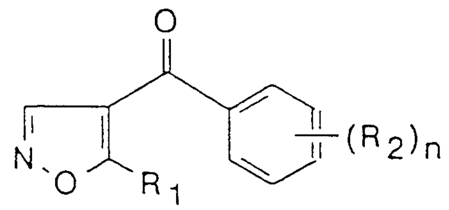

(II)

using, for example sodium borohydride in a polar solvent such as ethanol and at temperatures from 0° to 50°C. It is to be understood that functionality in either $R_1$ or $R_2$ which might be reactive to these conditions is to be selectively protected.

Furthermore, compounds of general formula (I) wherein $R_6$ is an -OH group and $R_7$ is an $R_5$ group may be prepared by reaction of a compound of general formula (II) with an appropriate organometallic reagent such as a Grignard reagent, in an inert solvent, such as ether or glyme and at temperatures from 0°C to the refluxing temperature of the solvent.

Compounds of general formula (I) may be converted into other compounds of general formula (I).

According to a further feature of the present invention, compounds of general formula (I) in which $R_6$ is $-CO_2R_5$ may be prepared from compounds of general formula (I) wherein $R_6$ is a cyano group by hydrolysis of the cyano group to the corresponding carboxylic acid which is subsequently esterified by known methods, for example by treatment with thionyl chloride followed by reaction with an alcohol of formula $R_5$-OH.

According to a further feature of the present invention compounds of general formula (I) wherein $R_6$ is an -OH group and $R_7$ is a hydrogen atom or $R_5$ group, may be converted into other compounds of general formula (I) wherein $R_6$ is an $-OCOR_8$ group, or an -OCO-phenyl-$(R_2)$q group, or an $-OCH_2$-phenyl-$(R_2)_q$ group, or an -O-phenyl-$(R_2)_q$ group, or an -OCO-Het1 group, or an $-OCONR_3R_4$ group, or an $-OSO_2R_8$ group, or an $-OSO_2$ -phenyl-$(R_2)$q group, or an $-OSO_2NR_3R_4$ group, and $R_7$ is a hydrogen atom or an $R_5$ group, by the reaction of a compound of general formula (I) in which $R_6$ is a hydroxy group and $R_7$ is hydrogen or an $R_5$ group, with the corresponding halogen compound in the presence of a suitable base such as pyridine in an inert solvent such as dichloromethane, and at temperatures from 0°C to the refluxing temperature of the solvent.

Compounds of general formula (I) wherein $R_6$ is a chlorine or bromine atom and $R_7$ is a hydrogen atom or $R_5$ group, may be prepared from compounds of general formula (I) wherein $R_6$ is a hydroxy group and $R_7$ is a hydrogen atom or an $R_5$ group, by reaction with a halogenating agent such as, for example phosphorous trichloride or phosphorous tribromide, in an inert solvent such as ether or dichloromethane, and at temperatures from 0°C to the refluxing temperature of the solvent.

Alternatively, compounds of general formula (I) wherein $R_6$ is a halogen atom or a cyano group and $R_7$ is a hydrogen or $R_5$ group may be prepared from compounds of general formula (I) in which $R_6$ is a hydroxyl group and $R_7$ is a hydrogen atom or $R_5$ group by first conversion of the hydroxyl group to a leaving group such as the mesylate or tosylate group followed by reaction with an alkali metal halide such as sodium iodide or cesium fluoride or reaction with a tetraalkylammonium halide, for example, tetra-n-butylammonium, or an alkali metal cyanide such as potassium cyanide.

According to a further feature of the present invention compounds of general formula (I) wherein $R_6$ is an $-OR_5$ group, or an $-SR_5$ group, or a group Het2, or an $-NR_3R_4$ group, may be prepared by substitution of the halogen atom of compounds of general formula (I) in which $R_6$ is a halogen atom, with a suitable nucleophile in an inert solvent, and at temperatures from 0 to 50°C.

According to a further feature of the present invention compounds of general formula (I) wherein $R_6$ is an $-SOR_5$ group or an $-SO_2R_5$ group may be prepared by oxidation of the corresponding compound of general formula (I) in which $R_6$ is an $-SR_5$ group using, for example meta-chloroperoxybenzoic acid in an inert solvent, and at temperatures from 0°C to the refluxing temperature of the solvent.

According to a further feature of the present invention, compounds of formula (I) in which $R_6$ is an $-OR_5$ group or an $-SR_5$ group and $R_7$ is an - $OR_5$ group or an $-SR_5$ group, or $R_6$ and $R_7$ represent a cyclic ketal or cyclic thioketal, may be prepared by treating compounds of general formula (II) with the appropriate alcohol or thiol in an inert solvent such as toluene in the presence of an acid catalyst such as para-toluenesulphonic acid at from room temperature to the boiling point of the solvent.

According to a further feature of the present invention, compounds of formula (I) wherein $R_6$ represents a ketone group $-COR_3$ in which $R_3$ excludes hydrogen may be prepared from the corresponding compound in which $R_6$ represents a cyano group by reaction with an organometallic reagent such as a Grignard reagent in an inert solvent such as ether or tetrahydrofuran from room temperature to the reflux temperature of the solvent.

According to a further feature of the present invention, compounds of formula (I) wherein $R_6$ represents an aldehyde group $-COR_3$ in which $R_3$ represents hydrogen may be prepared from the corresponding compound in wich $R_6$ represents a cyano group by reduction using diisobutylaluminium hydride in an inert solvent such as ether or tetrahydrofuran.

According to a further feature of the present invention, compounds of formula (I) in which $R_6$ represents a group $-NR_3COR_5$ may be prepared from the corresponding compound in which $R_6$ represents a group $-NHR_3$ by reaction with an acyl compound of formula $R_5CO-X$ wherein X represents a leaving group such as chlorine.

According to a further feature of the present invention, compounds of formula (I) in which $R_6$ represents an amide group, $-CONR_3R_4$ may be prepared from the corresponding compound in which $R_6$ represents an ester group $-CO_2R_5$ by acidic hydrolysis of said ester group followed by conversion of the carboxylic acid thus obtained to the corresponding acid chloride using for example thionyl chloride. The acid chloride may be converted to the amide by treatment with an amine of formula $H-NR_3R_4$.

According to a further feature of the present invention, compounds wherein $R_6$ represents a group $-CSNR_3R_4$ may be prepared by reacting compounds in which $R_6$ represents a group $-CONR_3R_4$ with Lawesson's reagent, [2,4-bis(4-methoxyphenyl)-1,3-dithia-2,4-diphosphetane-2,4-disulfide] as described in, for example Synthesis,941 (1979)

Intermediates in the preparation of compounds of general formula (I) are prepared by the application or adaptation of known methods.

Compounds of general formula (II) may be prepared by the reaction of a compound of general formula (III):

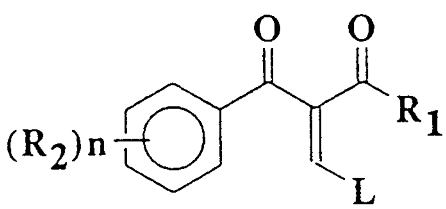

(III)

wherein L is a leaving group such as ethoxy or dimethylamino, with a salt of hydroxylamine, for example hydroxylamine hydrochloride in a polar solvent such as ethanol at the appropriate pH, and at temperatures from ambient to the refluxing temperature of the solvent.

Compounds of general formula (III) may be prepared as described by Schwan et al.,(J. Heterocyclic Chem., 1976, **13**, 973.) by for example, reacting the appropriate dione with for example, triethylorthoformate, in the presence of an acid catalyst, such as acetic anhydride.

Compounds of formula (III) in which L represents a dimethylamino group may be prepared by reacting the appropriate dione with a dimethylformamide dialkyacetal.

Compounds of general formula (II) may also be prepared by the reaction of a compound of general formula (IV):

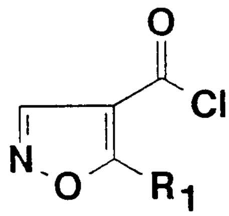

(IV)

with an appropriately substituted benzene in the presence of a Lewis acid catalyst such as aluminium chloride in an inert solvent such as dichloromethane and at temperatures from 0 to 50 ° C.

Compounds in which $R_2$ represents an $-SOR_5$ group or an $-SO_2R_5$ group may be prepared by oxidation of the corresponding compound in which $R_2$ represents an $-SR_5$ group using, for example meta-chloroperoxybenzoic acid in an inert solvent, and at temperatures from 0 ° C to the refluxing temperature of the solvent.

Compounds of general formula (IV) may be prepared as described by Doleschall and Seres [J. Chem. Soc. Perkin Trans. I,(1988),1875] or an adaptation thereof.

According to a further feature of the present invention, compounds of general formula (I) in which $R_6$ represents a group selected from $R_5$; an alkenyl group containing up to 6 carbon atoms which may be optionally substituted by one or more halogen atoms; or a cycloalkyl group containing from 3 to 6 carbon atoms which may be optionally substituted by one or more $R_5$ groups or one or more halogen atoms or a group $-COOR_5$;

may be prepared by reaction of a compound of general formula (V):

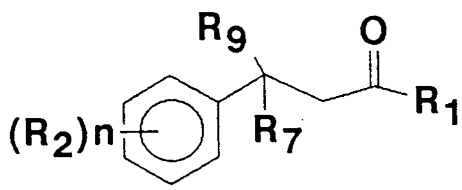

(V)

wherein $R_9$ represents a group selected from $R_5$; an alkenyl group containing up to 6 carbon atoms which may be optionally substituted by one or more halogen atoms; or a cycloalkyl group containing from 3 to 6 carbon atoms which may be optionally substituted by one or more $R_5$ groups or one or more halogen atoms or a group $-COOR_5$;

with a trialkylorthoformate, for example triethylorthoformate in the presence of an acid catalyst, such as acetic anhydride, followed by cyclisation to the desired isoxazole using a salt of hydroxylamine, optionally in the presence of a base such as triethylamine, in an inert solvent and at temperatures from 0 to 50 ° C.

The following examples illustrate the preparation of compounds of general formula (I). In the present specification, b.p. means boiling point, m.p. means melting point (average). When the letters NMR appear, this means that the characteristics of the proton nuclear magnetic resonance spectrum follow. If not otherwise specified the percentages are by weight.

## EXAMPLE 1

Compound a, b, c, h, j, k, l, o, p, v, w, x, y, z, aa, ab, ac, ad, ae, af, ag, ah.

A mixture of 4-[2-nitro-4-trifluoromethylbenzoyl]-5-cyclopropyl-isoxazole (8.00g) and sodium borohydride (0.53g) in ethanol (250ml) was stirred at room temperature for 2 hours. The solution was evaporated almost to dryness and the resultant solution was diluted with ethyl acetate. The solution was washed with water, dried (anhydrous magnesium sulphate) and filtered. The titrate was evaporated to dryness. The residue was purified by chromatography on silica eluted with a mixture of ether and petroleum ether to give 4-[hydroxy-(2-nitro-4-trifluoromethylphenyl)methyl]-5-cyclopropylisoxazole (5.95g) as an off

white solid: m.p. 76 ° C.

By proceeding in a similar manner the following compounds of general formula (I) were prepared:

| Compound No. | $R_1$ | $(R_2)_n$ | $R_6$ | $R_7$ | m.p. |
|---|---|---|---|---|---|
| b | Methyl | 2-$NO_2$-4-$CF_3$ | H | OH | 90° |
| c | Cyclopropyl | 2-Cl-3-OEt-4-$SO_2$Me | H | OH | * |

NMR: ($CDCl_3$) 1.0 (4H,m) 1.4 (3H,t) 2.1 (1H,m) 3.16 (3H,s) 3.6 (1H,br.s) 4.37 (2H,q) 6.1(1H,s) 7.69 (1H,m) 7.8 (1H,s) 7.81 (1H,m).

| Compound No. | $R_1$ | $(R_2)_n$ | $R_6$ | $R_7$ | m.p. |
|---|---|---|---|---|---|
| h | Cyclopropyl | 2-Cl-4-$SO_2$Me | H | OH | 141°C |
| j | Cyclopropyl | 2-$NO_2$-4-Me | H | OH | 82°C |
| k | Methyl | 2-Cl-3-OEt-4-$SO_2$Et | H | OH | * |

NMR: ($CDCl_3$) 1.2 (3H,t) 1.5 (3H,t) 2.6 (3H,s) 3.4 (2H,q) 4.3 (2H,q) 7.2 (1H,d) 8.0 (1H, d) 8.2 (1H,s).

| Compound No. | $R_1$ | $(R_2)_n$ | $R_6$ | $R_7$ | m.p. |
|---|---|---|---|---|---|
| l | Cyclopropyl | 2-Cl-3-OEt-4-$SO_2$Et | H | OH | * |

NMR: ($CDCl_3$) 1.2 (5H, m) 1.3 (2H,m) 1.5 (3H,t) 2.6 (1H,m) 3.5 (2H,q) 4.4 (2H,q) 7.3 (1H,d) 8.0 (1H,d) 8.2 (1H,s).

| | | | | | |
|---|---|---|---|---|---|
| o | Cyclopropyl | 2-Cl-4-F | H | OH | * |

NMR: ($CDCl_3$) 1.1 (2H,m) 1.3 (2H,m) 2.5 (1H,m) 7.0 (1H,m) 7.1 (1H,m) 7.3 (1H,m) 8.1 (1H,s).

| | | | | | |
|---|---|---|---|---|---|
| p | Cyclopropyl | 2-$CF_3$-4-$SO_2$Me | H | OH | 90°C |
| v | Cyclopropyl | 2-$SO_2$Me | H | OH | 118°C |
| w | 1-Methylcyclopropyl | 2,3-$Cl_2$-4-$SO_2$Me | H | OH | 205°C |
| x | Cyclopropyl | 2-$SO_2$Me-4-Cl | H | OH | 91.5°C |
| y | Cyclopropyl | 2-Br-4-$SO_2$Me | H | OH | 148°C |
| z | Cyclopropyl | 2-$SO_2$Me-4-Br | H | OH | 95°C |
| aa | Cyclopropyl | 2-$CH_3$-4-$SO_2$Me | H | OH | 136°C |
| ab | Cyclopropyl | 2-Cl-4-SMe | H | OH | 104°C |
| ac | Cyclopropyl | 2-$SO_2$Me-4-$CF_3$ | H | OH | 89°C |
| ad | Cyclopropyl | 2-Cl-4-$SOCH_3$ | H | OH | * |

NMR (DMSO): 0.8 (2H,m). 0.9 (2H,m). 2.2 (1H,m) 2.8 (3H,s) 6.1 (H,br.d) 6.3 (1H,br.d) 7.6 (2H,m) 8.0 (1H,m) 8.2 (1H, s).

| | | | | | |
|---|---|---|---|---|---|
| ae | Cyclopropyl | 2-$CF_3$-4-SMe | H | OH | 91°C |
| af | Cyclopropyl | 2-F-4-$SO_2$Me | H | OH | 101°C |
| ag | Isopropyl | 2-$SO_2$Me-4-Cl | H | OH | 145°C |
| ah | 1-Methylcyclopropyl | 2-$SO_2$Me-4-Cl | H | OH | 154°C |

**EXAMPLE 2**

Compound (d), (m), (q), and (u)

A mixture of 4-[hydroxy-(2-nitro-4-trifluoromethylphenyl)methyl]-5-cyclopropylisoxazole (0.69g), acetyl chloride (0.27g) and pyridine (0.10g) in dichloromethane (40ml) was stirred at 0°C for 4 hours. The mixture was quenched with water and extracted with dichloromethane. The organic extracts were dried (anhydrous magnesium sulphate) and filtered. The filtrate was evaporated to dryness.The residue was triturated with ether/petroleum ether to give 4-[acetoxy-(2-nitro-4-trifluoromethylphenyl)methyl]-5-cyclopropylisoxazole (0.46g) as a yellow solid: m.p. 82°C.

By proceeding in a similar manner the following compounds of formula (I) were prepared:

| Compound No. | $R_1$ | $(R_2)_n$ | $R_6$ | $R_7$ | m.p. |
|---|---|---|---|---|---|
| m | Cyclopropyl | 2-Cl-4-$SO_2$Me | H | -OCOMe | * |
| NMR ($CDCl_3$): 1.1 (2H,m) 1.2 (2H,m) 2.1 (3H,s) 2.2 (1H,m) 3.0 (3H,s) 7.1 (1H,s) 7.9 (3H,m) 8.0 (1H,s). | | | | | |
| q | Cyclopropyl | 2-$CF_3$-4-$SO_2$Me | H | -OCOMe | 149°C |
| u | Cyclopropyl | 2-$CF_3$-4-$SO_2$Me | H | -$OCOCF_3$ | 117°C |

**EXAMPLE 3**

Compound (e) and (t)

A mixture of 4-[hydroxy-(2-nitro-4-trifluoromethylphenyl)methyl]-5-cyclopropylisoxazole (0.89g) and phosphorous trichloride (0.57g) in dichloromethane (50ml) were stirred at 0°C for 2 hours. The reaction mixture was quenched with water and extracted with dichloromethane. The organic extracts were dried (anhydrous magnesium sulphate), filtered and evaporated to dryness. The crude product was purified by column chromatography on silica eluted with a mixture of petroleum ether/ethyl acetate to give 4-[chloro-(2-nitro-4-trifluoromethylphenyl)methyl]-5-cyclopropylisoxazole (0.79g) as a yellow oil: NMR: ($CDCl_3$) 1.1 (4H,m) 1.9(1H,m) 6.9(1H,s) 7.9(1H,m) 8.0(1H,s) 8.25(2H,m).

By proceeding in a similar manner the following compound was prepared: 4-[Chloro-(2-trifluoromethyl-4-methanesulphonylphenyl)methyl]-5-cyclopropylisoxazole: m.p. 145°C.

**EXAMPLE 4**

Compound (f) and (s)

A mixture of 4-[hydroxy-(2-nitro-4-trifluoromethylphenyl)methyl]-5-cyclopropylisoxazole (1.0g), phosphorous tribromide (0.70g) and pyridine (0.05g) in dry ether was stirred at 0°C for 1.5 hours. The reaction mixture was quenched with water and extracted with ether. The organic extracts were dried (anhydrous magnesium sulphate), filtered and the solvent evaporated yielding 4-[bromo-(2-nitro-4-trifluoromethylphenyl)methyl]-5-cyclopropylisoxazole (1.18g) as a yellow oil: NMR: (CDCl3) 1.1(4H,m) 1.9(1H,m) 6.9(1H,s) 7.9(1H,m) 8.0(1H,s) 8.15(2H,m).

By proceeding in a similar manner the following compounds were prepared:

4-[bromo-(2-trifluoromethyl-4-methanesulphonylphenyl)methyl]-5-cyclopropylisoxazole: m.p. 146°C.

4-[bromo-(2-chloro-4-methanesulphonylphenyl)methyl]-5-cyclopropylisoxazole: NMR ($CDCl_3$); 1.2-(4H,m), 1.9(1H,m), 3.1 (3H,s), 6.5 (1H,s), 7.9 (3H,m), 8.1 (1H,s).

**EXAMPLE 5**

Compound (g)

A mixture of 4-[bromo-(2-nitro-4-trifluoromethylphenyl)methyl]-5-cyclopropylisoxazole (1.2g) and dimethylamine (2.0g of a 33% solution in ethanol) in dichloromethane was stirred at 0°C for 2 hours and then at room temperature for a further 16 hours. The reaction mixture was quenched with water and extracted with dichloromethane. The organic extracts were dried (anhydrous magnesium sulphate), filtered

and evaporated to dryness. The residue was purified by column chromatography on silica eluting with petroleum ether and ethyl acetate to give 4-[N,N-dimethylamino-(2-nitro-4-trifluoromethylphenyl)methyl]-5-cyclopropyl-isoxazole (0.27g) as a yellow oil: NMR ($CDCl_3$); 1.02(4H,m), 2.08(1H,m), 2.12(6H,s), 4.9(1H,s), 7.8(1H,m), 7.9(1H,m), 8.0(1H,s), 8.1(1H,m).

**EXAMPLE 6**

Compounds (i), (n) and (r)

A mixture of 4-[bromo-(2-chloro-4-methanesulphonylphenyl)methyl]-5-cyclopropylisoxazole (2.5g) and methanol (2.0ml) in toluene was stirred at reflux for 16 hours. The resultant solution was evaporated to dryness and the residue was dissolved in ethyl acetate. This solution was washed with water, dried ($MgSO_4$), and filtered. The filtrate was evaporated to dryness. The residue was purified by chromatography on silica eluted with ethyl acetate and petroleum ether to give 4-[methoxy-(2-chloro-4-methanesulphonylphenyl)methyl]-5-cyclopropylisoxazole (1.40g) as a colourless oil. NMR: ($CDCl_3$) 1.0(4H,m), 2.1 (1H,m), 3.0-(3H,s), 3.3(3H,s), 5.6(1H,s), 7.9(4H,m).

By proceeding in a similar manner the following compounds were prepared:

4-[methoxy-(2-nitro-4-trifluoromethylphenyl)methyl]-5-cyclopropylisoxaszole; NMR ($CDCl_3$): 1.0(4H,m), 2.1(1H,s), 3.3(3H,s), 6.0(1H,s), 7.8(2H,m), 8.0(1H,d), 8.1(1H,s);

4-[methoxy-(2-trifluoromethyl-4-methanesulphonylphenyl)methyl]-5-cyclopropylisoxazole, m.p. 159 °C.

**REFERENCE EXAMPLE 1**

A mixture of crude 2-ethoxymethylene-1-(2-nitro-4-trifluoromethylphenyl)butan-1,3-dione (13.25g) and hydroxylamine hydrochloride (3.7g) in ethanol was stirred for 5 hours. The solution was evaporated almost to dryness and the resultant solution was diluted with ethyl acetate. The solution was washed with water, dried (anhydrous sodium sulphate) and filtered. The filtrate was evaporated to dryness. The residue was triturated with a mixture of ether/petroleum ether and the resultant buff solid was filtered off. The solid was dissolved in dichloromethane and filtered through silica. The silica was washed with dichloromethane and the combined filtrates were evaporated to dryness to give 4-[2-nitro-4-trifluoromethylbenzoyl]-5-methylisoxazole (4.5g) as an off-white solid, m.p. 86 °C.

By proceeding in a similar manner,the following compounds were prepared:

4-[2-Nitro-4-trifluoromethylbenzoyl]-5-cyclopropylisoxazole, as an off-white solid, m.p. 125 °C;

4-[2-chloro-3-ethoxy-4-methanesulphonylbenzoyl]-5-cyclopropylisoxazole as a white solid, m.p. 120 °C;

4-[2-nitro-4-trifluoromethylbenzoyl]-5-cyclopropylisoxazole, m.p. 125 °C.

**REFERENCE EXAMPLE 2**

A mixture of crude 2-ethoxymethylene-1-(2-chloro-4-methanesulphonylphenyl)-3-cyclopropyl propan-1,3-dione (6.85g), hydroxylamine hydrochloride (1.6g) and triethylamine (1.9g) in acetonitrile was stirred at room temperature for 16 hours. The solution was evaporated to dryness and the residue diluted with ethyl acetate. The resultant solution was washed with water, dried ($MgSO_4$) and filtered. The filtrate was evaporated to dryness. The residue was recrystallized from ethanol to give 4-[2-chloro-4-methanesulphonylbenzoyl]-5-cyclopropylisoxazole (2.4g) as a white solid, m.p. 115 °C.

By proceeding in a similar manner the following compounds were prepared.

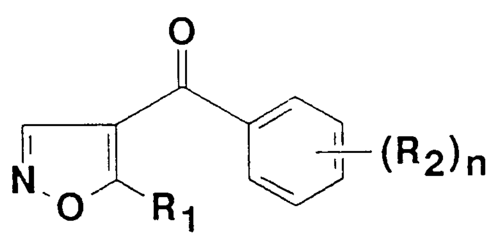

| $R_1$ | $(R_2)n$ | m.p. |
|---|---|---|
| Cyclopropyl | 2-Cl-3-OEt-4-$SO_2$Me | 120°C |
| Cyclopropyl | 2-$NO_2$-4-$CH_3$ | 54°C |
| Methyl | 2-Cl-3-OEt-4-$SO_2$Et | 109°C |
| Cyclopropyl | 2-Cl-3-OEt-4-$SO_2$Et | 128°C |
| Cyclopropyl | 2-Cl-4-F | 83°C |
| Cyclopropyl | 2-$CF_3$-4-$SO_2$Me | 146°C |

**REFERENCE EXAMPLE 3**

A mixture of 1-(2-trifluoromethyl-4-(methanethio)phenyl)-2-ethoxymethylene-3-cyclopropylpropan-1,3-dione (14.9g), hydroxylamine hydrochloride (3.16g) and sodium acetate (3.75g) in ethanol were stirred for 16 hours. The resulting suspension was evaporated to dryness. The residue was diluted with ethyl acetate and water. The layers were separated and the organic layer was washed with water, dried ($MgSO_4$) and filtered. The filtrate was evaporated to dryness. The residue was purified by chromatography on silica eluted with a mixture of ethyl acetate and petrolium ether to give 4-[2-trifluoromethyl-4-(methanethio)-benzoyl]-5-cyclopropylisoxazole (8.6g) as a white solid, m.p. 63 ° C.

By proceeding in a similar manner the following compounds of general formula (II) were prepared:

| $R_1$ | $(R_2)n$ | m.p. |
|---|---|---|
| Cyclopropyl | 2-$SO_2$Me | 119°C |
| Cyclopropyl | 2-Br-4-$SO_2$Me | 110°C |
| 1-Methylcyclopropyl | 2,3-$Cl_2$-4-$SO_2$Me | 86°C |
| Cyclopropyl | 2-$SO_2$Me-4-Cl | 182°C |
| Cyclopropyl | 2-$SO_2$Me-4-Br | 193°C |
| Cyclopropyl | 2-$CH_3$-4-$SO_2$Me | 87°C |
| Cyclopropyl | 2-Cl-4-SMe | * |
| NMR: ($CDCl_3$) | 1.1(2H,m), 1.2(2H,m), 2.4(3H,s), 2.5(1H,m), 7.0(1H,m), 7.1(1H,d), 7.2(1H,d), 8.2(1H,s). | |
| Isopropyl | 2-$SO_2$Me-4-Cl | 134°C |
| 1-methylcyclopropyl | 2-$SO_2$Me-4-Cl | 136°C |
| Cyclopropyl | 2-F-4-$SO_2$Me | 116°C |
| Cyclopropyl | 2-$SO_2$Me-4-$CF_3$ | 134°C |

## REFERENCE EXAMPLE 4

4-(2-chloro-4-(methanethio)benzoyl)-5-cyclopropylisoxazole (2.0g) in dichloromethane was stirred at -15°C and to this was added metachloroperoxybenzoic acid (MCPBA) (2.0g). The mixture was stirred at -15°C for 1 hour and then at room temperature for 16 hours. The solution was washed with 1M sodium bisulphite, water, dried ($MgSO_4$) and filtered. The filtrate was evaporated to dryness and purified by chromatography on silica eluting with ethyl acetate and petroleum spirit to give 4-(2-chloro-4-methanesulphinylbenzoyl)-5-cyclopropylisoxazole as a white solid, m.p. 117°C.

## REFERENCE EXAMPLE 5

A mixture of 1-(2-nitro-4-trifluoromethylphenyl)-butan-1,3-dione (11.0g), triethyl orthoformate (11.3g) and acetic anhydride (12.3g) was stirred and heated at reflux for 3 hours. After cooling, the mixture was evaporated to dryness and toluene was added. The mixture was evaporated to dryness to give 2-ethoxymethylene-1-(2-nitro-4-trifluoromethylphenyl)-butan-1,3-dione as a crude brown oil which was not further purified.

By proceeding in a similar manner, the following compounds were prepared:

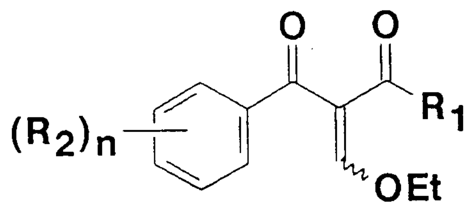

| $R_1$ | $(R_2)_n$ |
|---|---|
| Cyclopropyl | $2-NO_2-4-CF_3$ |
| Cyclopropyl | $2-Cl-3-OEt-4-SO_2Me$ |
| Cyclopropyl | $2-Cl-4-SO_2Me$ |
| Cyclopropyl | $2-NO_2-4-Me$ |
| Methyl | $2-Cl-3-OEt-4-SO_2Et$ |
| Cyclopropyl | $2-Cl-3-OEt-4-SO_2Et$ |
| Cyclopropyl | 2-Cl-4-F |
| Cyclopropyl | $2-CF_3-4-SO_2Me$ |
| Cyclopropyl | $2-SO_2Me$ |
| 1-Methylcyclopropyl | $2,3-Cl_2-4-SO_2Me$ |
| Cyclopropyl | $2-SO_2Me-4-Cl$ |
| Cyclopropyl | $2-Br-4-SO_2Me$ |
| Cyclopropyl | $2-SO_2Me-4-Br$ |
| Cyclopropyl | $2-CH_3-4-SO_2Me$ |
| Cyclopropyl | 2-Cl-4-SMe |
| Cyclopropyl | $2-CF_3-4-SMe$ |
| Cyclopropyl | $2-SO_2Me-4-CF_3$ |
| Cyclopropyl | $2-F-4-SO_2Me$ |
| Isopropyl | $2-SO_2Me-4-Cl$ |
| 1-Methylcyclopropyl | $2-SO_2Me-4-Cl$ |

## REFERENCE EXAMPLE 6

A mixture of crude t-butyl 2-(2-nitro-4-trifluoromethylbenzoyl)-3-oxobutanoate (9.1g) and 4-toluenesulphonic acid (0.1g) in dry toluene was stirred and heated at reflux for 3 hours. The cooled mixture was extracted with aqueous sodium hydroxide solution and then with water. The aqueous extracts were acidified to pH 1 and extracted with ether. The combined organic layers were washed with water, dried (anhydrous sodium sulphate) and filtered. The filtrate was evaporated to dryness and the residue was recrystallized from petroleum ether to give 1-(2-nitro-4-trifluoromethylphenyl)-butan-1,3-dione (4.6g) as an off-white solid, m.p. 81 °C.

By proceeding in a similar manner, the following compounds were prepared:

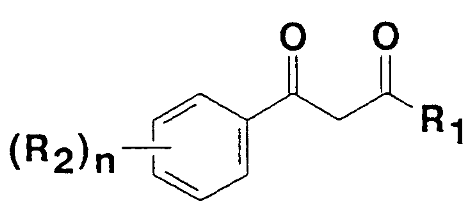

| $R_1$ | $(R_2)n$ | m.p. |
|---|---|---|
| Cyclopropyl | $2\text{-}NO_2\text{-}4\text{-}CF_3$ | 96° |
| Cyclopropyl | $2\text{-}Cl\text{-}3\text{-}OEt\text{-}4\text{-}SO_2Me$ | * |
| NMR: ($CDCl_3$) 1.1(4H,m) 1.3(3H,t) 1.9(1H,m) 3.1(3H,s) 4.2(2H,q) 5.95(1H,s) 6.25(1H,m) 6.65(1H,m) 14.9(1H,br s). | | |
| Cyclopropyl | $2\text{-}Cl\text{-}4\text{-}SO_2Me$ | 93°C |
| Cyclopropyl | $2\text{-}NO_2\text{-}4\text{-}Me$ | * |

NMR: ($CDCl_3$) 1.1(4H,m) 2.0(1H,m) 2.5(3H,s) 6.6(1H,s) 8.5(2H,m) 8.7(1H,m).

| | | |
|---|---|---|
| Methyl | 1-Cl-3-OEt-4-$SO_2$Et | * |

NMR: ($CDCl_3$) 1.2-1.9 (6H,m) 2.3(3H,s) 3.55(2H,q) 4.4(2H,q) 6.0(1H,s) 7.45(1H,d) 7.95(1H,d).

| | | |
|---|---|---|
| Cyclopropyl | 2-Cl-3-OEt-4-$SO_2$Et | * |

NMR: ($CDCl_3$) 1.2-2.3(11H,m) 3.75(2H,q) 4.6(2H,q) 6.3(1H,s) 7.2(1H,d) 8.2(1H,d).

| | | |
|---|---|---|
| Cyclopropyl | 2-Cl-4-F | not purified |
| Cyclopropyl | 2-$CF_3$-4-$SO_2$Me | not purified |
| Cyclopropyl | 2-$SO_2$Me | 94°C |
| 1-Methylcyclopropyl | 2,3-$Cl_2$-4-$SO_2$Me | 136°C |
| Cyclopropyl | 2-$SO_2$Me-4-Cl | * |

NMR: ($CDCl_3$) 1.1(4H,m) 2.1(1H,m) 3.3(3H,s) 5.8(1H,s) 7.3-7.9(3H,m).

| | | |
|---|---|---|
| Cyclopropyl | 2-Br-4-$SO_2$Me | 109°C |
| Cyclopropyl | 2-$SO_2$Me-4-Br | not purified |
| Cyclopropyl | 2-$CH_3$-4-$SO_2$Me | not purified |
| Cyclopropyl | 2-Cl-4-SMe | * |

NMR: ($CDCl_3$) 0.8-2.2(4H,m) 1.6(1H,m) 2.4(3H,s) 6.0(1H,s) 6.9-7.4(3H,m) 15.o(1H,br s).

| | | |
|---|---|---|
| Cyclopropyl | 2-$CF_3$-4-SMe | * |

NMR: ($CDCl_3$) 1.1(4H,m) 1.7(1H,m) 2.5(3H,s) 5.8(1H,s) 7.1-7.5(3H,m) 14.5(1H br s).

| | | |
|---|---|---|
| Cyclopropyl | 2-$SO_2$Me-4-$CF_3$ | * |

NMR ($CDCl_3$) 1.2(4H,m) 2.4(1H,m) 3.3(3H,s) 5.9(1H,s) 7.1-8.1(3H,m).

| | | |
|---|---|---|
| Cyclopropyl | 2-F-4-$SO_2$Me | not purified |
| Isopropyl | 2-$SO_2$Me-4-Cl | * |

NMR ($CDCl_3$): 1.2(6H,d) 2.3(1H,m) 3.3(3H,s) 5.7(1H,s) 7.0-7.9 (3H,m).

| | | |
|---|---|---|
| 1-Methylcyclopropyl | 2-$SO_2$Me-4-Cl | * |

NMR (CDCl3): 0.8(2H,m) 1.2(5H,m) 3.3(3H,s) 5.8(1H,s) 7.0-8.0(3H,m).

## REFERENCE EXAMPLE 7

A mixture of magnesium turnings (4.8g) and carbon tetrachloride (2ml) in pure ethanol was stirred and warmed gently to 50°C until the reaction was initiated (effervescence observed). Ether was added cautiously with stirring. A solution of t-butyl 3-oxobutanoate (31.6g) in ether was added dropwise at such a rate as to maintain the mixture at reflux. Stirring and heating at reflux was continued for 2 hours. A solution of 2-nitro-4-trifluoromethylbenzoyl chloride (50.7g) in ether was added dropwise and the resultant solution was stirred and heated at reflux for 2.5 hours. The cooled reaction mixture was treated with hydrochloric acid with stirring and the two layers were separated. The organic phase was extracted with aqueous sodium hydroxide solution and water. The combined aqueous layers were acidified to pH 1 and extracted with ether. The combined organic layers were washed with water, dried (anhydrous magnesium sulphate) and filtered. The filtrate was evaporated to dryness to give t-butyl 2-(2-nitro-4-trifluoromethylbenzoyl)-3-oxo butanoate (62.2g) as a crude red oil which was not further purified.

By proceeding in a similar manner, the following compounds were prepared:

t-Butyl 2-(2-nitro-4-trifluoromethylbenzoyl)-3-oxo-3-cyclopropylpropanoate, as a crude orange oil; and

t-Butyl 2-(2-chloro-3-ethoxy-4-methanesulphonylbenzoyl)-3-oxo-3-cyclopropyl-propanoate, as a yellow oil.

## REFERENCE EXAMPLE 8

Carbon tetrachloride (1ml) was added to a stirred mixture of t-butyl 3-cyclopropyl-3-oxopropanoate (7.83g) and Magnesium (1.08g) in methanol causing a vigorous reaction The reaction mixture was stirred for 0.25 hours and evaporated to dryness. The residue was suspended in acetonitrile and a solution of 2-chloro-4-(methanethio)benzoyl chloride (9.38g) in acetonitrile was added dropwise. Stirring was continued at room temperature for 4 hours. The resultant solution was evaporated to dryness and the residue dissolved in ethyl acetate, washed with 2M HCl solution followed by water. The ethyl acetate solution was dried ($MgSO_4$) and filtered. The filtrate was evaporated to dryness to give t-butyl 2-[2-chloro-4-(methanethio)-benzoyl]-3-cyclopropyl-3-oxopropanoate (15.8g) as an orange oil which was not purified further.

By proceeding in a similar manner the following compounds were prepared:

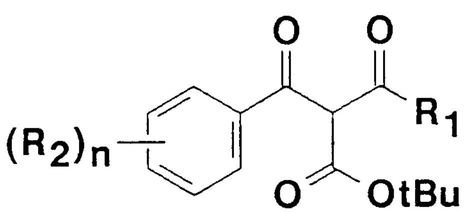

| $R_1$ | $(R_2)_n$ |
|---|---|
| Cyclopropyl | 2-$NO_2$-4-CF3 |
| Cyclopropyl | 2-Cl-3-OEt-4-$SO_2$Me |
| Cyclopropyl | 2-Cl-4-$SO_2$Me |
| Cyclopropyl | 2-$NO_2$-4-Me |
| Methyl | 2-Cl-3-OEt-4-$SO_2$Me |
| Cyclopropyl | 2-Cl-3-OEt-4-$SO_2$Me |
| Cyclopropyl | 2-Cl-4-F |
| Cyclopropyl | 2-$CF_3$-4-$SO_2$Me |
| Cyclopropyl | 2-$SO_2$Me |
| 1-Methylcyclopropyl | 2,3-$Cl_2$-4-$SO_2$Me |
| Cyclopropyl | 2-$SO_2$Me-4-Cl |
| Cyclopropyl | 2-Br-4-$SO_2$Me |
| Cyclopropyl | 2-$SO_2$Me-4-Br |
| Cyclopropyl | 2-$CH_3$-4-$SO_2$Me |
| Cyclopropyl | 2-$CF_3$-4-SMe |
| Cyclopropyl | 2-$SO_2$Me-4-$CF_3$ |
| Cyclopropyl | 2-F-4-$SO_2$Me |
| Isopropyl | 2-$SO_2$Me-4-Cl |
| 1-Methylcyclopropyl | 2-$SO_2$Me-4-Cl |

**REFERENCE EXAMPLE 9**

A mixture of 5-cyclopropylcarbonyl-2,2-dimethyl-1,3-dioxan-4,6-dione (23.9g) and t-butanol (25g) in dry toluene was stirred and heated at 80°C for 4 hours. The cooled mixture was washed with water, dried (anhydrous sodium sulphate), treated with decolourizing charcoal and filtered. The filtrate was evaporated to dryness to give t-butyl 3-cyclopropyl-3-oxopropanoate (20.2g) as an orange oil; NMR: ($CDCl_3$) 0.8(2H,m), 0.89(2H,m), 1.35(9H,s), 1.9(1H,m), 3.35(2H,s).

**REFERENCE EXAMPLE 10**

A mixture of 2,2-dimethyl-1,3-dioxan-4,6-dione (20.0g) and pyridine (22.0g) in dichloromethane was stirred at 0°C. A solution of cyclopropylcarbonyl chloride (16.0g) in dichloromethane (50ml) was added dropwise under an inert atmosphere whilst maintaining the temperature below 3°C. The mixture was stirred at 0°C for 1 hour and at ambient temperature for 2 hours. The resultant orange suspension was washed with hydrochloric acid, water, dried (anhydrous sodium sulphate) and filtered. The filtrate was evaporated to dryness. The residue was dissolved in ether and the solution was treated with decolourizing charcoal and filtered. The filtrate was evaporated to dryness to give 5-cyclopropylcarbonyl-2,2-dimethyl-1,3-dioxan-4,6-dione (24.2g) as a yellow solid, m.p. 45°C.

**REFERENCE EXAMPLE 11**

A solution of n-butyl lithium (2.5m in hexane, 36ml) was added dropwise with stirring to a cooled solution of diisopropylamine (9.09g) in dry tetrahydrofuran under an inert atmosphere, whilst maintaining the temperature below 70°C. The mixture was stirred for 0.25 hours and t-butyl trimethylsilylacetate (16.9g) was added dropwise with stirring whilst maintaining the temperature below 70°C. The mixture was stirred at -78°C for 1 hour. A suspension of 1-(1-methylcyclopropylcarbonyl)-imidazole (13.5g) in dry THF was added dropwise whilst maintaining the temperature below -60°C. The mixture was stirred at -78°C for 3 hours and the temperature was allowed to rise to room temperature. 2M hydrochloric acid solution was added cautiously and the mixture was extracted with ether. The combined extracts were washed with 2M hydrochloric acid solution and water, dried (anhydrous sodium sulphate) and filtered. The filtrate was evaporated to dryness to give t-butyl 3-(1-methylcyclopropyl)-3-oxopropionate as an orange oil, NMR: ($CDCl_3$) 0.65(2H,m), 1.1(2H,m), 1.25(3H,s), 1.4(9H,s), 3.2(2H,s).

**REFERENCE EXAMPLE 12**

A solution of 1-methylcyclopropylcarbonyl chloride (11.8g) in toluene was added dropwise to a solution of imidazole (13.6g) in tetrahydrofuran whilst maintaining the temperature below 25°C. The mixture was stirred for 3 hours and filtered. The filtrate was evaporated to dryness to give 1-methylcyclopropylcarbonyl)-imidazole (13.6g) as a white solid, m.p. 35°C.

According to a feature of the present invention, there is provided a method for controlling the growth of weeds (i.e. undesired vegetation) at a locus which comprises applying to the locus a herbicidally effective amount of at least one isoxazole derivative of general formula (I) or an agriculturally acceptable salt thereof. For this purpose, the isoxazole derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:

broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and

grass weeds, for example Alopecurus myosuroides, Avena fatua, Digitaria sanguinalis, Echinochloa crus-galli, Eleusine indica and Setaria spp, e.g. Setaria faberii or Setaria viridis, and

sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 20kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after planting of the crop or before or after emergence of the crop. For the selective control of weeds at a locus of weed infestation which is an area used, or to be used, for growing of crops, e.g. the crops hereinbefore mentioned, application rates between 0.01kg and 8.0kg, and preferably between 0.01kg and 4.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25kg and 10.0kg, and preferably between 0.5kg and 8.0kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise

dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the isoxazole derivatives of general formula I or an agriculturally acceptable salt thereof, in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, absorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid

concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are

aqueous suspension concentrates which comprise from 10 to 70% of one or more compounds of general formula (I), from 2 to 10% of surface-active agent, from 0.1 to 5% of thickener and from 15 to 87.9% of water;

wettable powders which comprise from 10 to 90% of one or more compounds of general formula (I), from 2 to 10% of surface-active agent and from 8 to 88% of solid diluent or carrier;

water soluble or water dispersible powders which comprise from 10 to 90% of one or more compounds of general formula (I), from 2 to 40% of sodium carbonate and from 0 to 88% of solid diluent;

liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water;

liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;

granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and

emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6′-diethyl-N-(methoxymethyl)-acetanilide], atrazine [2-chloro-4-ethylamino-6-isopropylamino-1,3,5-triazine], bromoxynil [3,5-dibromo-4-hydroxybenzonitrile], chlortoluron [N′-(3-chloro-4-methylphenyl)-N,N-dimethylurea], cyanazine [2-chloro-4-[1-cyano-1-methylethylamino)-6-ethylamino-1,3,5-triazine], 2,4-D [2,4-dichlorophenoxyacetic acid], dicamba [3,6-dichloro-2-methoxybenzoic acid], difenzoquat [1,2-dimethyl-3,5-diphenyl-pyrazolium salts], flampropmethyl [methyl N-2-(N-benzoyl-3-chloro-4-fluoroanilino)-propionate], fluometuron [N′-(3-trifluoromethylphenyl)-N,N-dimethylurea], isoproturon [N′-(4-isopropylphenyl)-N,N-dimethylurea], insecticides, e.g. synthetic pyrethroids, e.g. permethrin and cypermethrin, and fungicides, e.g. carbamates, e.g. methyl N-(1-butyl-carbamoyl-benzimidazol-2-yl)-carbamate, and triazoles e.g. 1-(4-chloro-phenoxy)-3,3-dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the isoxazole derivatives of general formula (I) or, as is preferred, a herbicidal composition as hereinbefore described, and preferably a herbicidal concentrate which must be diluted before use, comprising at least one of the isoxazole derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to

control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

**EXAMPLE C1**

A wettable powder was formed from:

| | |
|---|---|
| * active ingredient (compound (a)): | 50% w/w |
| * nonylphenol/ethylene oxide condensate containing 9 moles of ethylene oxide per mol of phenol: | 5% w/w |
| * silicon dioxide of microfine particle size: | 5% w/w |
| * synthetic magnesium silicate carrier: | 40% w/w |

by absorbing the condensate on the silicon dioxide, mixing with the other ingredients and grinding the mixture in a hammermill to give a wettable powder.

Similar wettable powders may be prepared as described above by replacing the isoxazole (compound (a)) by other compounds of general formula (I).

**EXAMPLE C2**

An aqueous suspension concentrate was formed from:

| | |
|---|---|
| * active ingredient (compound (a)): | 50% w/v |
| * nonylphenol/ethylene oxide condensate containing 9 moles of ethylene oxide per mol of phenol: | 1 % w/v |
| * sodium salt of polycarboxylic acid: | 0.2% w/v |
| * Ethylene glycol: | 5% w/v |
| * polysaccharide xanthan gum thickener: | 0.15% w/v |
| * water to | 100% by volume |

by intimately mixing the ingredients and grinding in a ball-mill for 24 hours.

Similar aqueous concentrates may be prepared as described above by replacing the isoxazole (compound (a)) by other compounds of general formula (I).

Representative compounds of general formula (I) have been used in herbicidal applications or use according to the following procedures.

**METHOD OF USE OF HERBICIDAL COMPOUNDS:**

a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare.

b) Weed control : Pre-emergence

The seeds were sown in 70 mm square, 75 mm deep plastic pots in non-sterile soil. The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |
| 2) Grass weeds | |
| Alopecurus myosuroides | 15 |
| Avena fatua | 10 |
| Echinochloa crus-galli | 15 |
| Setaria viridis | 20. |
| 3) Sedges | |
| Cyperus esculentus | 3. |
| Crop | |
| 1) Broad-leafed | |
| Cotton | 3 |
| Soya | 3. |
| 2) Grass | |
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead. Visual assessment of crop damage was made 20-24 days after spraying. The results were expressed as the percentage reduction in growth or damage to the crop or weeds, in comparison with the plants in the control pots.

c) Weed control : Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-

| 1) Broad leafed weeds | | |
|---|---|---|
| Weed species | Number of plants per pot | Growth stage |
| Abutilon theophrasti | 3 | 1-2 leaves |
| Amaranthus retroflexus | 4 | 1-2 leaves |
| Galium aparine | 3 | $1^{st}$ whorl |
| Ipomoea purpurea | 3 | 1-2 leaves |
| Sinapis arvensis | 4 | 2 leaves |
| Xanthium strumarium | 1 | 2-3 leaves. |

| 2) Grass weeds | | |
|---|---|---|
| Weed species | Number of plants per pot | Growth stage |
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

| 3) Sedges | | |
|---|---|---|
| Weed species | Number of plants per pot | Growth stage |
| Cyperus esculentus | 3 | 3 leaves. |

| 1) Broad leafed | | |
|---|---|---|
| Crops | Number of plants per pot | Growth stage |
| Cotton | 2 | 1 leaf |
| Soya | 2 | 2 leaves. |

| 2) Grass | | |
|---|---|---|
| Crops | Number of plants per pot | Growth stage |
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

The compounds of the invention, used at 4kg/ha or less, have shown an excellent level of herbicidal activity together with crop tolerance on the weeds used in the foregoing experiments.

When applied pre-emergence at 4000g/ha compounds (a, e, and o) gave at least 90% reduction in growth of one or more of the weed species.

When applied post-emergence at 4000g/ha compounds (a, e and o) gave at least 90% reduction in growth of one or more of the weed species.

When applied pre-emergence at 2000g/ha compound (b) gave at least 90% reduction in growth of one or more of the weed species.

When applied post-emergence at 2000g/ha compound (b) gave at least 90% reduction in growth of one or more of the weed species.

When applied pre-emergence at 1000g/ha compounds (c, d, g, h, i, j, k, l, m, n, p, q, r, s, t, u, v, w, x, y, z, aa, ab, ac, ad, ae, af and ah) gave at least 90% reduction in growth of one or more of the weed species.

When applied post-emergence at 1000g/ha compounds (c, d, h, i, j, k, l, m, n, p, r, s, t, u, w, x, y, z, aa, ab, ac, ad, ae, af and ah) gave at least 90% reduction in growth of one or more of the weed species.

## Claims

### Claims for the following Contracting States : AT, BE, CH, LI, DE, DK, FR, GB, GR, IT, LU, NL, SE

**1.** 4-Benzyl isoxazole derivatives of general formula (I):

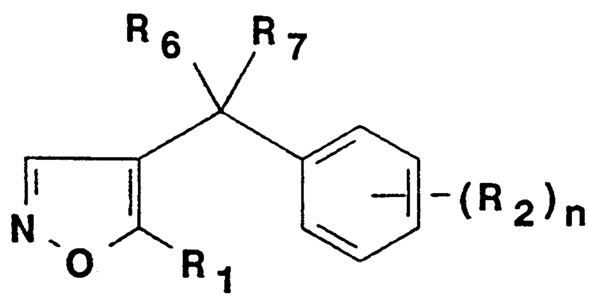

(I)

wherein $R_1$ represents:

a straight- or branched- chain alkyl, alkenyl or alkynyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$; or

a cycloalkenyl group containing 5 or 6 carbon atoms which may be optionally substituted by one or more $R_5$ groups or one or more halogen atoms or a group $COOR_5$; or

an aryl or aralkyl group of general formula $[(R_2)_q$ phenyl$]-[C(R_3)(R_4)]_p-$ (aryl is generally C6-C10 and aralkyl is generally C7-C11); or

an ester group, $COOR_5$; or

an aldehyde or acyl group, $COR_3$; or

a cyano or nitro group; or

an amino group, $-NR_3R_4$; or

a halogen atom;

$R_2$ represents:

a nitro or cyano group; or

a halogen atom; or

a group, $R_5$; or

a sulphenyl, sulphinyl or sulphonyl group, $-S(O)_mR_5$; or

a sulphamoyl group, $-SO_2NR_3R_4$; or

an ester group, $-COOR_5$; or

an acyl or aldehyde group, $-COR_3$; or

a carbamoyl or thiocarbamoyl group $-CONR_3R_4$ or $-CSNR_3R_4$; or

an alkoxy group, $-OR_5$; or

an alkyl group (with 1 to 3 carbon atoms) substituted by $-OR_5$;

$R_3$ and $R_4$, which may be the same or different, each represents:

a hydrogen atom or a straight- or branched- chain alkyl group containing up to 6 carbon atoms which is optionally substituted by one or more halogen atoms;

$R_5$ represents:

a straight- or branched-chain alkyl group containing up to 6 carbon atoms which is optionally substituted by one or more halogen atoms;

$R_6$ represents:

a hydrogen atom; or

a hydroxy group, OH; or

a halogen atom; or

a group $R_5$; or

an alkenyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$; or

a sulphenyl, sulphinyl or sulphonyl group, $-S(O)_mR_5$; or

an ester group, $-COOR_5$; or

a cyano group; or

an acyl or aldehyde group, $-COR_3$; or

a carbamoyl or thiocarbamoyl group, $-CONR_3R_4$ or $-CSNR_3R_4$;

or

an alkoxy group, $-OR_5$; or

a phenoxy group, -O-phenyl-$(R_2)$q; or

a benzyloxy group, $-OCH_2$-phenyl-$(R_2)$q; or

an acyloxy group, $-OCOR_8$; or

a benzoyloxy group, -OCO-phenyl-$(R_2)$q; or

a group -OCO-Het1; or

a carbamoyloxy group, $-OCONR_3R_4$; or

an alkylsulphonyloxy group, $-OSO_2R_8$; or

a phenylsulphonyloxy group, $-OSO_2$-phenyl-$(R_2)$q

a sulphamoyloxy group, $-OSO_2NR_3R_4$; or

an amino group, $-NR_3R_4$; or

an acylamino group, $-NR_3COR_5$; or

a group Het2;

$R_7$ represents:

a hydrogen atom; or

a group, $R_5$;

or $R_6$ and $R_7$ may form with the carbon atom to which they are attached, a ketal $((OR_5)_2)$, or a thioketal $((SR_5)_2)$, or a cyclic ketal or cyclic thioketal containing 5 or 6 atoms in the ring optionally substituted by one or more $R_5$ groups;

$R_8$ represents:

a straight- or branched- chain alkyl or alkenyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$;

Het1 represents a heterocycle containing 5 or 6 atoms in the ring, one or more of which is a heteroatom selected from nitrogen, sulphur and oxygen, the ring carbon atoms being optionally substituted by a group $R_2$;

Het2 represents a heterocyclic group selected from: pyrrol-l-yl, pyrazol-l-yl, imidazol-l-yl, 1,2,4-triazol-4-yl, 1,2,4-triazoyl-l-yl, 1,2,3-triazol-l-yl or 1,2,3-triazol-2-yl; each of which may be optionally substituted by one or more groups $R_2$;

m represents zero, 1, or 2;

n represents an integer from 1 to 5;

q represents zero or an integer from 1 to 5;

p represents zero or 1;

and agriculturally acceptable salts thereof.

**2.** A compound according to claim 1 wherein:

a) one of the groups represented by $(R_2)_n$ is in the ortho position on the phenyl ring; and/or

b) where there are two groups represented by $(R_2)_n$ they are in the 2-and 4- positions on the phenyl ring; and/or

c) $R_1$ is alkyl optionally substituted by halogen; or cycloalkyl optionally substituted by alkyl or halogen; ; and/or

d) $R_2$ is halogen; or nitro; or $R_5$; or $-S(O)_mR_5$; or $-OR_5$; or alkyl substituted by $-OR_5$; or $-COOR_5$; and/or

e) $R_5$ is alkyl (with 1 to 3 carbon atoms) optionally substituted by fluorine or chlorine; and/or

f) $R_6$ is hydrogen, halogen, -OH, $-OR_5$, $-OCOR_8$, or alkyl (with one to four carbon atoms) optionally substituted by one or more halogen atoms; and/or

g) One of the groups $R_6$ or $R_7$ is a hydrogen atom; and/or

h) $R_7$ is hydrogen or an alkyl group containing from one to four carbon atoms; and/or

i) $R_8$ is an alkyl group containing from one to four carbon atoms optionally substituted by one or more halogen atoms; and/or

j) n is 2 or 3.

**3.** A compound according to claim 1 or 2 which is:

4-[hydroxy-(2-nitro-4-trifluoromethylphenyl)methyl]-5-cyclopropylisoxazole;
4-[hydroxy-(2-nitro-4-trifluoromethylphenyl)methyl]-5-methyl isoxazole;
4-[hydroxy-(2-chloro-3-ethoxy-4-methanesulphonylphenyl)methyl]-5-cyclopropylisoxazole;
4-[acetoxy-(2-nitro-4-trifluoromethylphenyl)methyl]-5-cyclopropylisoxazole;
4-[chloro-(2-nitro-4-trifluoromethylphenyl)methyl]-5-cyclopropylisoxazole;
4-[bromo-(2-nitro-4-trifluoromethylphenyl)methyl]-5-cyclopropylisoxazole;
4-[N,N-dimethylamino-(2-nitro-4-trifluoromethylphenyl)methyl]-5-cyclopropylisoxazole;
4-[hydroxy-(2-chloro-4-methanesulphonylphenyl)methyl]-5-cyclopropylisoxazole;
4-[methoxy-(2-nitro-4-trifluoromethylphenyl)methyl]-5-cyclopropylisoxazole;
4-[hydroxy-(2-nitro-4-methylphenyl)methyl]-5-cyclopropylisoxazole;
4-[hydroxy-(2-chloro-3-ethoxy-4-ethanesulphonylphenyl)methyl]-5-methylisoxazole;
4-[hydroxy-(2-chloro-3-ethoxy-4-ethanesulphonylphenyl)methyl]-5-cyclopropylisoxazole;
4-[acetoxy-(2-chloro-4-methanesulphonylphenyl)methyl]-5-cyclopropylisoxazole;
4-[methoxy-(2-chloro-4-methanesulphonylphenyl)methyl]-5-cyclopropylisoxazole;
4-[hydroxy-(2-chloro-4-fluorophenyl)methyl]-5-cyclopropylisoxazole;
4-[hydroxy-(2-trifluoromethyl-4-methanesulphonylphenyl)methyl]-5-cyclopropylisoxazole;
4-[acetoxy-(2-trifluoromethyl-4-methanesulphonylphenyl)methyl]-5-cyclopropylisoxazole;
4-[methoxy-(2-trifluoromethyl-4-methanesulphonylphenyl)methyl]-5-cyclopropylisoxazole;
4-[bromo-(2-trifluoromethyl-4-methanesulphonylphenyl)methyl]-5-cyclopropylisoxazole;
4-[chloro-(2-trifluoromethyl-4-methanesulphonylphenyl)methyl]-5-cyclopropylisoxazole;
4-[trifluoroacetoxy-(2-trifluoromethyl-4-methanesulphonylphenyl) methyl]-5-cyclopropylisoxazole;
4-[hydroxy-(2-methanesulphonylphenyl)methyl]-5-cyclopropylisoxazole;
4-[hydroxy-(2,3-dichloro-4-methanesulphonylphenyl)methyl]-5-(1-methylcyclopropyl)isoxazole;
4-[hydroxy-(2-methanesulphonyl-4-chlorophenyl)methyl]-5-cyclopropylisoxazole;
4-[hydroxy-(2-bromo-4-methanesulphonylphenyl)methyl]-5-cyclopropylisoxazole;
4-[hydroxy-(2-methanesulphonyl-4-bromophenyl)methyl]-5-cyclopropylisoxazole;
4-[hydroxy-(2-methyl-4-methanesulphonylphenyl)methyl]-5-cyclopropylisoxazole;
4-[hydroxy-(2-chloro-4-methanethiophenyl)methyl]-5-cyclopropylisoxazole;
4-[hydroxy-(2-methanesulphonyl-4-fluoromethylphenyl)methyl]-5-cyclopropylisoxazole;
4-[hydroxy-(2-chloro-4-methanesulphinylphenyl)methyl]-5-cyclopropylisoxazole;
4-[hydroxy-(2-trifluoromethyl-4-methanethiophenyl)methyl]-5-cyclopropylisoxazole;
4-[hydroxy-(2-fluoro-4-methanesulphonylphenyl)methyl]-5-cyclopropylisoxazole;
4-[hydroxy-(2-methanesulphonyl-4-chlorophenyl)methyl]-5-isopropylisoxazole; or
4-[hydroxy-(2-methanesulphonyl-4-chlorophenyl)methyl]-5-(1-methylcyclopropyl)isoxazole.

**4.** A process for the prepararation of a compound accordig to claim 1 which comprises:

a) where $R_6$ is an OH group and $R_7$ represents the hydrogen atom, the reduction of a compound of general formula (II):

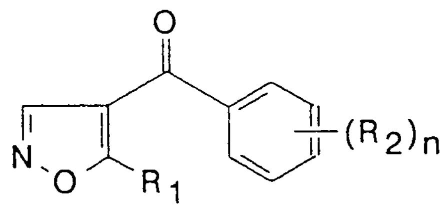

(II)

in which the other symbols are as defined in claim 1;

b) where $R_6$ is an -OH group and $R_7$ is an $R_5$ group, the reaction of a compound of general formula (II) with an appropriate organometallic reagent in an inert solvent;

c) where $R_6$ is an $-OCOR_8$ group, or an -OCO-phenyl-$(R_2)q$ group, or an $-OCH_2$-phenyl-$(R_2)_q$ group, or an -O-phenyl-$(R_2)_q$ group, or an -OCO-Het1 group, or an $-OCONR_3R_4$ group, or an $-OSO_2R_8$ group, or an $-OSO_2$-phenyl-$(R_2)q$ group, or an $-OSO_2NR_3R_4$ group, and $R_7$ is a hydrogen atom or an $R_5$ group,

the reaction of a compound of general formula (I) in which $R_6$ is a hydroxy group and $R_7$ is hydrogen or an $R_5$ group, with the corresponding halogen compound in the presence of a suitable base;

d) where $R_6$ is a chlorine or bromine atom and $R_7$ is a hydrogen atom or $R_5$ group, the reaction of a compound of general formula (I) wherein $R_6$ is a hydroxy group and $R_7$ is a hydrogen atom or an $R_5$ group,with a halogenating agent;

e) where $R_6$ is a halogen atom or a cyano group and $R_7$ is a hydrogen or $R_5$ group, by first converting the hydroxyl group $R_6$ of a compound of general formula (I) in which $R_6$ is a hydroxyl group and $R_7$ is a hydrogen atom or $R_5$ group to a leaving group followed by reaction with an alkali metal halide, a tetraalkylammonium halide or an alkali metal cyanide;

f) where $R_6$ is an $-OR_5$ group, or an $-SR_5$ group, or a group Het2, or an $-NR_3R_4$ group, the substitution of the halogen atom of a compound of general formula (I) in which $R_6$ is a halogen atom, with a suitable nucleophile in an inert solvent;.

g) where $R_6$ is an $-SOR_5$ group or an $-SO_2R_5$ group, the oxidation of the corresponding compound of general formula (I) in which $R_6$ is an $-SR_5$ group;

h) where $R_6$ is an $-OR_5$ group or an $-SR_5$ group and $R_7$ is an $-OR_5$group or an $-SR_5$ group, or $R_6$ and $R_7$ represent a cyclic ketal or cyclic thioketal, the treatment of compounds of general formula (II) with the appropriate alcohol or thiol in an inert solvent in the presence of an acid catalyst;

i) where $R_6$ represents a group $-COR_{3,}$ in which $R_3$ excludes hydrogen, the reaction of the corresponding compound in which $R_6$ represents a cyano group with an organometallic reagent in an inert solvent;

j) where $R_6$ represents an aldehyde group $-COR_3$ in which $R_3$ represents hydrogen, the reduction of the corresponding compound in which $R_6$ represents a cyano group using diisobutylaluminium hydride;

k) where $R_6$ represents a group $-NR_3COR_5$; the reaction of the corresponding compound in which $R_6$ represents a group $-NHR_3$ with an acyl compound of formula $R_5CO$-X wherein X represents a leaving group;

l) where $R_6$ represents an amide group, $-CONR_3R_4$, the acidic hydrolysis of the corresponding compound in which $R_6$ represents an ester group $-CO_2R_5$ followed by conversion of the carboxylic acid thus obtained to the corresponding acid chloride; followed by conversion to the amide by treatment with an amine of formula $H-NR_3R_4$.

m) where $R_6$ represents a group $-CSNR_3R_4$, reacting compounds in which $R_6$ represents a group $-CONR_3R_4$ with Lawesson's reagent, [2,4-bis(4-methoxyphenyl)-1,3-dithia-2,4-diphosetane-2,4-disulfide];

n) where $R_6$ represents a group selected from $R_5$; an alkenyl group containing up to 6 carbon atoms which may be optionally substituted by one or more halogen atoms; or a cycloalkyl group containing from 3 to 6 carbon atoms which may be optionally substituted by one or more $R_5$ groups or one or more halogen atoms or a group $-COOR_5$;

by reaction of a compound of general formula (V):

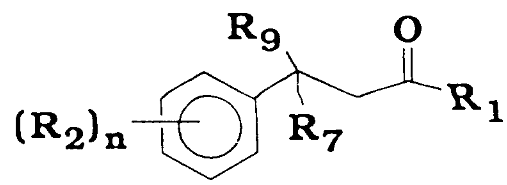

(V)

wherein $R^9$ represents a group selected from $R_5$; an alkenyl group containing up to 6 carbon atoms which may be optionally substituted by one or more halogen atoms; or a cycloalkyl group containing from 3 to 6 carbon atoms which may be optionally substituted by one or more $R_5$ groups or one or more halogen atoms or a group $-COOR_5$;

with a trialkylorthoformate in the presence of an acid catalyst using a salt of hydroxylamine;

o) where $R_6$ is $-CO_2R_5$, by hydrolysis of the cyano group of the corresponding compound of general formula (I) in which $R_6$ is a cyano group to the corresponding carboxylic acid which is subsequently esterified;

and optionally converting the compound of formula (I) thus obtained into an agriculturally acceptable salt thereof.

**5.** A herbicidal composition which comprises as active ingredient a herbicidally effective amount of a 4-benzyl isoxazole according to any one of claims 1 to 3 or an agriculturally acceptable salt thereof, in association with an agriculturally acceptable diluent or carrier and/or surface active agent.

**6.** A herbicidal composition according to claim 5 which comprises 0.05 to 90% by weight of above ingredient.

**7.** A herbicidal composition according to claim 5 or 6 which is in liquid form and contains from 0.05 to 25% of surface-active agent.

**8.** A herbicidal compositions according to any one of claims 5 to 7 in the form of an aqueous suspension concentrate, a wettable powder, a water soluble or water dispersible powder, a liquid water soluble concentrate, a liquid emulsifiable suspension concentrate, a granule or an emulsifiable concentrate.

**9.** A method for controlling the growth of weeds at a locus which comprises applying to the locus a herbicidally effective amount of a 4-benzyl isoxazole derivative according to any one of claims 1 to 3 or an agriculturally acceptable salt thereof.

**10.** A method according to claim 9 in which the locus is an area used, or to be used, for growing of crops and the compound is applied at an application rate from 0.01 kg to 4.0 kg per hectare.

**11.** A method according to claim 9 in which the locus is an area which is not a crop-growing area and the compound is applied at an application rate from 1.0 kg to 20.0 kg per hectare.

**12.** An article of manufacture comprising at least one isoxazole derivative according to any one of claims 1 to 3 or a herbicidal composition according to any one of claims 5 to 8, and preferably a herbicidal concentrate which must be diluted before use, comprising at least one of the isoxazole derivatives within a container for the aforesaid derivative or derivatives, or a said herbicidal composition and instructions physically associated with the aforesaid container setting out the manner in which the aforesaid derivative or derivatives or herbicidal composition contained therein is to be used to control the growth of weeds.

**Claims for the following Contracting State : ES**

1. A process for the preparation of a 4-benzyl isoxazole derivative of general formula (I):

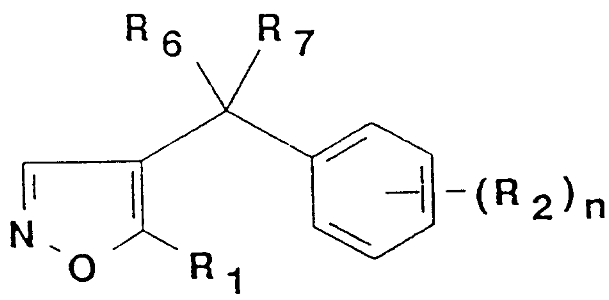

(I)

wherein $R_1$ represents:

a straight- or branched- chain alkyl, alkenyl or alkynyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$; or

a cycloalkenyl group containing 5 or 6 carbon atoms which may be optionally substituted by one or more $R_5$ groups or one or more halogen atoms or a group $COOR_5$; or

an aryl or aralkyl group of general formula $[(R_2)_q$ phenyl$]-[C(R_3)(R_4)]_p-$ (aryl is generally C6-C10 and aralkyl is generally C7-C11); or

an ester group, $COOR_5$; or

an aldehyde or acyl group, $COR_3$; or

a cyano or nitro group; or

an amino group, $-NR_3R_4$; or

a halogen atom;

$R_2$ represents:

a nitro or cyano group; or

a halogen atom; or

a group, $R_5$; or

a sulphenyl, sulphinyl or sulphonyl group, $-S(O)_mR_5$; or

a sulphamoyl group, $-SO_2NR_3R_4$; or

an ester group, $-COOR_5$; or

an acyl or aldehyde group, $-COR_3$; or

a carbamoyl or thiocarbamoyl group $-CONR_3R_4$ or $-CSNR_3R_4$; or

an alkoxy group, $-OR_5$; or

an alkyl group (with 1 to 3 carbon atoms) substituted by $-OR_5$;

$R_3$ and $R_4$, which may be the same or different, each represents:

a hydrogen atom or a straight- or branched- chain alkyl group containing up to 6 carbon atoms which is optionally substituted by one or more halogen atoms;

$R_5$ represents:

a straight- or branched-chain alkyl group containing up to 6 carbon atoms which is optionally substituted by one or more halogen atoms;

$R_6$ represents:

a hydrogen atom; or

a hydroxy group, OH; or

a halogen atom; or

a group $R_5$; or

an alkenyl group containing up to 6 carbon atoms which may be optionally substituted by one or more halogen atoms; or

a cycloalkyl group containing from 3 to 6 carbon atoms which may be optionally substituted by one or more $R_5$ groups or one or more halogen atoms or a group $-COOR_5$; or

a sulphenyl, sulphinyl or sulphonyl group, $-S(O)_mR_5$; or

an ester group, $-COOR_5$; or
a cyano group; or
an acyl or aldehyde group, $-COR_3$; or
a carbamoyl or thiocarbamoyl group, $-CONR_3R_4$ or $-CSNR_3R_4$;
or
an alkoxy group, $-OR_5$; or
a phenoxy group, -O-phenyl-$(R_2)_q$; or
a benzyloxy group, $-OCH_2$-phenyl-$(R_2)$q; or
an aryloxy group, $-OCOR_8$; or
a benzoyloxy group, -OCO-phenyl-$(R_2)$q; or
a group -OCO-Het1; or
a carbamoyloxy group, $-OCONR_3R_4$; or
an alkylsulphonyloxy group, $-OSO_2R_8$; or
a phenylsulphonyloxy group, $-OSO_2$-phenyl-$(R_2)$q
a sulphamoyloxy group, $-OSO_2NR_3R_4$; or
an amino group, $-NR_3R_4$; or
an acylamino group, $-NR_3COR_5$; or
a group Het2;
$R_7$ represents:
a hydrogen atom; or
a group, $R_5$; or $R_6$ and $R_7$ may form with the carbon atom to which they are attached, a ketal (-$(OR_5)_2$), or a thioketal ($(SR_5)_2$), or a cyclic ketal or cyclic thioketal containing 5 or 6 atoms in the ring optionally substituted by one or more $R_5$ groups;
$R_8$ represents:
a straight- or branched- chain alkyl or alkenyl group containing up to 6 carbon atoms which may be optionally substituted by one or more halogen atoms; or
a cycloalkyl group containing from 3 to 6 carbon atoms which may be optionally substituted by one or more $R_5$ groups or one or more halogen atoms or a group $-COOR_5$;
Het1 represents a heterocycle containing 5 or 6 atoms in the ring, one or more of which is a heteroatom selected from nitrogen, sulphur and oxygen, the ring carbon atoms being optionally substituted by a group $R_2$;
Het2 represents a heterocyclic group selected from: pyrrol-l-yl, pyrazol-l-yl, imidazol-l-yl, 1,2,4-triazol-4-yl, 1,2,4-triazoyl-l-yl, 1,2,3-triazol-l-yl or 1,2,3-triazol-2-yl; each of which may be optionally substituted by one or more groups $R_2$;
m represents zero, 1, or 2;
n represents an integer from 1 to 5;
q represents zero or an integer from 1 to 5;
p represents zero or 1;
or an agriculturally acceptable salt thereof,
which process comprises:
a) where $R_6$ is an OH group and $R_7$ represents the hydrogen atom, the reduction of a compound of general formula (II):

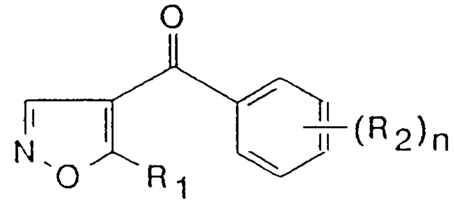

(II)

in which the other symbols are as hereinbefore defined;
b) where $R_6$ is an -OH group and $R_7$ is an $R_5$ group, the reaction of a compound of general formula (II) with an appropriate organometallic reagent in an inert solvent;

c) where $R_6$ is an $-OCOR_8$ group, or an -OCO-phenyl-$(R_2)q$ group, or an $-OCH_2$-phenyl-$(R_2)_q$ group, or an -O-phenyl-$(R_2)_q$ group, or an -OCO-Het1 group, or an $-OCONR_3R_4$ group, or an $-OSO_2R_8$ group, or an $-OSO_2$ -phenyl-$(R_2)q$ group, or an $-OSO_2NR_3R_4$ group, and $R_7$ is a hydrogen atom or an $R_5$ group,

the reaction of a compound of general formula (I) in which $R_6$ is a hydroxy group and $R_7$ is hydrogen or an $R_5$ group, with the corresponding halogen compound in the presence of a suitable base:

d) where $R_6$ is a chlorine or bromine atom and $R_7$ is a hydrogen atom or $R_5$ group, the reaction of a compound of general formula (I) wherein $R_6$ is a hydroxy group and $R_7$ is a hydrogen atom or an $R_5$ group,with a halogenating agent;

e) where $R_6$ is a halogen atom or a cyano group and $R_7$ is a hydrogen or $R_5$ group, by first converting the hydroxyl group $R_6$ of a compound of general formula (I) in which $R_6$ is a hydroxyl group and $R_7$ is a hydrogen atom or $R_5$ group to a leaving group followed by reaction with an alkali metal halide, a tetraalkylammonium halide or an alkali metal cyanide;

f) where $R_6$ is an $-OR_5$ group, or an $-SR_5$ group, or a group Het2, or an $-NR_3R_4$ group, the substitution of the halogen atom of a compound of general formula (I) in which $R_6$ is a halogen atom, with a suitable nucleophile in an inert solvent;

g) where $R_6$ is an $-SOR_5$ group or an $-SO_2R_5$ group, the oxidation of the corresponding compound of general formula (I) in which $R_6$ is an $-SR_5$ group;

h) where $R_6$ is an $-OR_5$ group or an $-SR_5$ group and $R_7$ is an $-OR_5$group or an $-SR_5$ group, or $R_6$ and $R_7$ represent a cyclic ketal or cyclic thioketal, the treatment of compounds of general formula (II) with the appropriate alcohol or thiol in an inert solvent in the presence of an acid catalyst;

i) where $R_6$ represents a group $-COR_3$, in which $R_3$ excludes hydrogen, the reaction of the corresponding compound in which $R_6$ represents a cyano group with an organometallic reagent in an inert solvent;

j) where $R_6$ represents an aldehyde group $-COR_3$ in which $R_3$ represents hydrogen, the reduction of the corresponding compound in which $R_6$ represents a cyano group using diisobutylaluminium hydride;

k) where $R_6$ represents a group $-NR_3COR_5$; the reaction of the corresponding compound in which $R_6$ represents a group $-NHR_3$ with an acyl compound of formula $R_5CO$-X wherein X represents a leaving group;

l) where $R_6$ represents an amide group, $-CONR_3R_4$, the acidic hydrolysis of the corresponding compound in which $R_6$ represents an ester group $-CO_2R_5$ followed by conversion of the carboxylic acid thus obtained to the corresponding acid chloride; followed by conversion to the amide by treatment with an amine of formula $H-NR_3R_4$.

m) where $R_6$ represents a group $-CSNR_3R_4$, reacting compounds in which $R_6$ represents a group $-CONR_3R_4$ with Lawesson's reagent, [2,4-bis(4-methoxyphenyl)-1,3-dithia-2,4-diphosetane-2,4-disulfide];

n) where $R_6$ represents a group selected from $R_5$; an alkenyl group containing up to 6 carbon atoms which may be optionally substituted by one or more halogen atoms; or a cycloalkyl group containing from 3 to 6 carbon atoms which may be optionally substituted by one or more $R_5$ groups or one or more halogen atoms or a group $-COOR_5$;

by reaction of a compound of general formula (V):

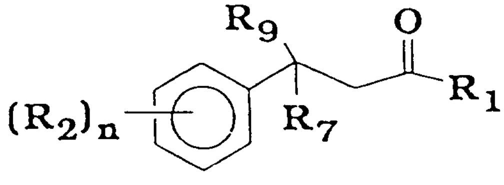

(V)

wherein $R^9$ represents a group selected from $R_5$; an alkenyl group containing up to 6 carbon atoms which may be optionally substituted by one or more halogen atoms; or a cycloalkyl group containing from 3 to 6 carbon atoms which may be optionally substituted by one or more $R_5$ groups

or one or more halogen atoms or a group $-COOR_5$;

with a trialkylorthoformate in the presence of an acid catalyst using a salt of hydroxylamine;

o) where $R_6$ is $-CO_2R_5$, by hydrolysis of the cyano group of the corresponding compound of general formula (I) in which $R_6$ is a cyano group to the corresponding carboxylic acid which is subsequently esterified;

and optionally converting the compound of formula (I) thus obtained into an agriculturally acceptable salt thereof.

**2.** A process according to claim 1 for the preparation of a compound of general formula (I) wherein:

a) one of the groups represented by $(R_2)_n$ is in the ortho position on the phenyl ring; and/or

b) where there are two groups represented by $(R_2)n$ they are in the 2-and 4- positions on the phenyl ring; and/or

c) $R_1$ is alkyl optionally substituted by halogen; or cycloalkyl optionally substituted by alkyl or halogen;; and/or

d) $R_2$ is halogen; or nitro; or $R_5$; or $-S(O)_mR_5$; or $-OR_5$; or alkyl substituted by $-OR_5$; or $-COOR_5$; and/or

e) $R_5$ is alkyl (with 1 to 3 carbon atoms) optionally substituted by fluorine or chlorine; and/or

f) $R_6$ is hydrogen, halogen, -OH, $-OR_5$, $-OCOR_8$, or alkyl (with one to four carbon atoms) optionally substituted by one or more halogen atoms; and/or

g) One of the groups $R_6$ or $R_7$ is a hydrogen atom; and/or

h) $R_7$ is hydrogen or an alkyl group containing from one to four carbon atoms; and/or

i) $R_8$ is an alkyl group containing from one to four carbon atoms optionally substituted by one or more halogen atoms; and/or

j) n is 2 or 3.

**3.** A process according to claim 1 or 2 for the preparation of a compound of general formula (I) which is:

4-[hydroxy-(2-nitro-4-trifluoromethylphenyl)methyl]-5-cyclopropylisoxazole;
4-[hydroxy-(2-nitro-4-trifluoromethylphenyl)methyl]-5-methyl isoxazole;
4-[hydroxy-(2-chloro-3-ethoxy-4-methanesulphonylphenyl)methyl]-5-cyclopropylisoxazole;
4-[acetoxy-(2-nitro-4-trifluoromethylphenyl)methyl]-5-cyclopropylisoxazole;
4-[chloro-(2-nitro-4-trifluoromethylphenyl)methyl]-5-cyclopropylisoxazole;
4-[bromo-(2-nitro-4-trifluoromethylphenyl)methyl]-5-cyclopropylisoxazole;
4-[N,N-dimethylamino-(2-nitro-4-trifluoromethylphenyl)methyl]-5-cyclopropylisoxazole;
4-[hydroxy-(2-chloro-4-methanesulphonylphenyl)methyl]-5-cyclopropylisoxazole;
4-[methoxy-(2-nitro-4-trifluoromethylphenyl)methyl]-5-cyclopropylisoxazole;
4-[hydroxy-(2-nitro-4-methylphenyl)methyl]-5-cyclopropylisoxazole;
4-[hydroxy-(2-chloro-3-ethoxy-4-ethanesulphonylphenyl)methyl]-5-methylisoxazole;
4-[hydroxy-(2-chloro-3-ethoxy-4-ethanesulphonylphenyl)methyl]-5-cyclopropylisoxazole;
4-[acetoxy-(2-chloro-4-methanesulphonylphenyl)methyl]-5-cyclopropylisoxazole;
4-[methoxy-(2-chloro-4-methanesulphonylphenyl)methyl]-5-cyclopropylisoxazole;
4-[hydroxy-(2-chloro-4-fluorophenyl)methyl]-5-cyclopropylisoxazole;
4-[hydroxy-(2-trifluoromethyl-4-methanesulphonylphenyl)methyl]-5-cyclopropylisoxazole;
4-[acetoxy-(2-trifluoromethyl-4-methanesulphonylphenyl)methyl]-5-cyclopropylisoxazole;
4-[methoxy-(2-trifluoromethyl-4-methanesulphonylphenyl)methyl]-5-cyclopropylisoxazole;
4-[bromo-(2-trifluoromethyl-4-methanesulphonylphenyl)methyl]-5-cyclopropylisoxazole;
4-[chloro-(2-trifluoromethyl-4-methanesulphonylphenyl)methyl]-5-cyclopropylisoxazole;
4-[trifluoroacetoxy-(2-trifluoromethyl-4-methanesulphonylphenyl) methyl]-5-cyclopropylisoxazole;
4-[hydroxy-(2-methanesulphonylphenyl)methyl]-5-cyclopropylisoxazole;
4-[hydroxy-(2,3-dichloro-4-methanesulphonylphenyl)methyl]-5-(1-methylcyclopropyl)isoxazole;
4-[hydroxy-(2-methanesulphonyl-4-chlorophenyl)methyl]-5-cyclopropylisoxazole;
4-[hydroxy-(2-bromo-4-methanesulphonylphenyl)methyl]-5-cyclopropylisoxazole;
4-[hydroxy-(2-methanesulphonyl-4-bromophenyl)methyl]-5-cyclopropylisoxazole;
4-[hydroxy-(2-methyl-4-methanesulphonylphenyl)methyl]-5-cyclopropylisoxazole;
4-[hydroxy-(2-chloro-4-methanethiophenyl)methyl]-5-cyclopropylisoxazole;
4-[hydroxy-(2-methanesulphonyl-4-trifluoromethylphenyl)methyl]-5-cyclopropylisoxazole;
4-[hydroxy-(2-chloro-4-methanesulphinylphenyl)methyl]-5-cyclopropylisoxazole;
4-[hydroxy-(2-trifluoromethyl-4-methanethiophenyl)methyl]-5-cyclopropylisoxazole;
4-[hydroxy-(2-fluoro-4-methanesulphonylphenyl)methyl]-5-cyclopropylisoxazole;

4-[hydroxy-(2-methanesulphonyl-4-chlorophenyl)methyl]-5-isopropylisoxazole; or
4-[hydroxy-(2-methanesulphonyl-4-chlorophenyl)methyl]-5-(1-methylcyclopropyl)isoxazole.

**4.** A process for the preparation of a herbicidal composition which comprises formulating, as active ingredient, a herbicidally effective amount of a 4-benzyl isoxazole as defined in any one of claims 1 to 3 or an agriculturally acceptable salt thereof, with an agriculturally acceptable diluent or carrier and/or surface active agent.

**5.** A process according to claim 4 wherein the herbicidal composition comprises 0.05 to 90% by weight of active ingredient.

**6.** A process according to claim 4 to 5 wherein the herbicidal composition is in liquid form and contains from 0.05 to 25% of surface-active agent.

**7.** A process according to any one of claims 4 to 6 wherein the herbicidal composition is in the form of an aqueous suspension concentrate, a wettable powder, a water soluble or water dispersible powder, a liquid water soluble concentrate, a liquid emulsifiable suspension concentrate, a granule or an emulsifiable concentrate.

**8.** A method for controlling the growth of weeds at a locus which comprises applying to the locus a herbicidally effective amount of a 4-benzyl isoxazole derivative as defined in any one of claims 1 to 3 or an agriculturally acceptable salt thereof.

**9.** A method according to claim 8 in which the locus is an area used, or to be used, for growing of crops and the compound is applied at an application rate from 0.01 kg to 4.0 kg per hectare.

**10.** A method according to claim 8 in which the locus is an area which is not a crop-growing area and the compound is applied at an application rate from 1.0 kg to 20.0 kg per hectare.

**11.** A process for the preparation of an article of manufacture comprising at least one isoxazole derivative as defined in any one of claims 1 to 3 or a herbicidal composition as defined in any one of claims 4 to 7, and preferably a herbicidal concentrate which must be diluted before use, which process comprises containing the said isoxazole derivative or derivatives or the said herbicidal composition in a container and placing in physical association with the aforesaid container instructions setting out the manner in which the aforesaid derivative or derivatives or herbicidal composition contained therein is to be used to control the growth of weeds.

**Patentansprüche**

**Patentansprüche für folgende Vertragsstaaten : AT, BE, CH, LI, DE, DK, FR, GB, GR, IT, LU, NL, SE**

**1.** 4-Benzylisoxazolderivate der allgemeinen Formel (I):

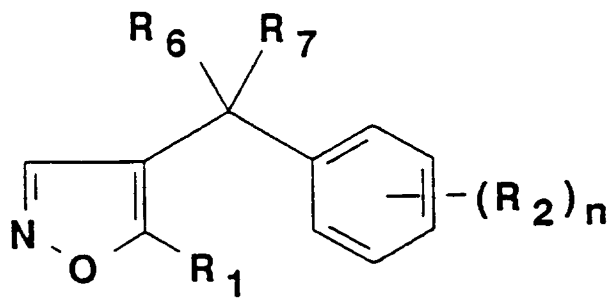

(I)

in der $R_1$ steht für:

eine gerad- oder verzweigtkettige Alkyl-, Alkenyl- oder Alkinylgruppe, die bis zu 6 Kohlenstoffatome enthält und die gegebenenfalls durch ein oder mehrere Halogenatom(e) substituiert sein kann, oder

eine Cycloalkylgruppe, die 3 bis 6 Kohlenstoffatome enthält und die gegebenenfalls durch eine oder mehrere Gruppe(n) $R_5$ oder ein oder mehrere Halogenatom(e) oder eine Gruppe $-COOR_5$ substituiert sein kann, oder

eine Cycloalkenylgruppe, die 5 oder 6 Kohlenstoffatome enthält und die gegebenenfalls durch eine oder mehrere Gruppe(n) $R_5$ oder ein oder mehrere Halogenatom(e) oder eine Gruppe $-COOR_5$ substituiert sein kann, oder

eine Aryl- oder Arylalkylgruppe der allgemeinen Formel $[(R_2)_q phenyl]-[C(R_3)(R_4)]_p-$ (wobei Aryl im allgemeinen C6-C10 und Arylalkyl im allgemeinen C7-C11 ist) oder

eine Estergruppe, $-COOR_5$, oder

eine Aldehyd- oder Acylgruppe, $-COR_3$, oder

eine Cyano- oder Nitrogruppe oder

eine Amingruppe, $-NR_3R_4$, oder

ein Halogenatom;

$R_2$ steht für:

eine Nitro- oder Cyanogruppe oder

ein Halogenatom oder

eine Gruppe $R_5$ oder

eine Sulfenyl-, Sulfinyl- oder Sulfonylgruppe, $-S(O)_mR_5$, oder

eine Sulfamoylgruppe, $-SO_2NR_3R_4$, oder

eine Estergruppe, $-COOR_5$, oder

eine Acyl- oder Aldehydgruppe, $-COR_3$, oder

eine Carbamoyl- oder Thiocarbamoylgruppe, $-CONR_3R_4$ oder $-CSNR_3R_4$, oder

eine Alkoxygruppe, $-OR_5$, oder

eine Alkylgruppe (mit 1 bis 3 Kohlenstoffatomen), die durch $-OR_5$ substituiert ist;

$R_3$ und $R_4$, die gleich oder unterschiedlich sein können, jeweils stehen für:

ein Wasserstoffatom oder eine gerad- oder verzweigtkettige Alkylgruppe, die bis zu 6 Kohlenstoffatome enthält und die gegebenenfalls durch ein oder mehrere Halogenatom(e) substituiert ist;

$R_5$ steht für:

eine gerad- oder verzweigtkettige Alkylgruppe, die bis zu 6 Kohlenstoffatome enthält und die gegebenenfalls durch ein oder mehrere Halogenatom(e) substituiert ist;

$R_6$ steht für:

ein Wasserstoffatom oder

eine Hydroxygruppe, OH, oder

ein Halogenatom oder

eine Gruppe $R_5$ oder

eine Alkenylgruppe mit bis zu 6 Kohlenstoffatomen, die gegebenenfalls durch ein oder mehrere Halogenatom(e) substituiert sein kann, oder

eine Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe $-COOR_5$ substituiert sein kann, oder

eine Sulfenyl-, Sulfinyl- oder Sulfonylgruppe, $-S(O)_mR_5$, oder

eine Estergruppe, $-COOR_5$, oder

eine Cyanogruppe oder

eine Acyl- oder Aldehydgruppe, $-COR_3$, oder

eine Carbamoyl- oder Thiocarbamoylgruppe, $-CONR_3R_4$ oder $-CSNR_3R_4$, oder

eine Alkoxygruppe, $-OR_5$, oder

eine Phenoxygruppe, $-O-phenyl-(R_2)_q$, oder

eine Benzyloxygruppe, $-OCH_2-phenyl-(R_2)_q$, oder

eine Acyloxygruppe, $-OCOR_8$, oder

eine Benzoyloxygruppe, $-OCO-phenyl-(R_2)_q$, oder

eine Gruppe -OCO-Het1 oder

eine Carbamoyloxygruppe, $-OCONR_3R_4$, oder

eine Alkylsulfonyloxygruppe, $-OSO_2R_8$, oder

eine Phenylsulfonyloxygruppe, $-OSO_2-phenyl-(R_2)_q$,

eine Sulfamoyloxygruppe, $-OSO_2NR_3R_4$, oder

eine Aminogruppe, $-NR_3R_4$, oder

eine Acylaminogruppe, $-NR_3COR_5$ oder

eine Gruppe Het2;

$R_7$ steht für:

ein Wasserstoffatom oder

eine Gruppe $R_5$;

oder $R_6$ und $R_7$ mit dem Kohlenstoffatom, an das sie gebunden sind, ein Ketal $((OR_5)_2)$ oder ein Thioketal $((SR_5)_2)$ oder ein cyclisches Ketal oder cyclisches Thioketal mit 5 oder 6 Atomen im Ring, gegebenenfalls substituiert durch eine oder mehrere Gruppe(n) $R_5$, bilden können;

$R_8$ steht für:

eine gerad- oder verzweigtkettige Alkyl- oder Alkenylgruppe mit bis zu 6 Kohlenstoffatomen, die gegebenenfalls durch ein oder mehrere Halogenatom(e) substituiert sein kann, oder

eine Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe $-COOR_5$ substituiert sein kann;

wobei Het1 für einen Heterocyclus mit 5 oder 6 Atomen im Ring steht, von denen eines oder mehrere ein Heteroatom, ausgewählt aus Stickstoff, Schwefel und Sauerstoff, ist bzw. sind, wobei die Ringkohlenstoffatome gegebenenfalls durch eine Gruppe $R_2$ substituiert sind;

wobei Het2 für eine heterocyclische Gruppe steht, ausgewählt aus Pyrrol-1-yl, Pyrazol-1-yl, Imidazol-1-yl, 1,2,4-Triazol-4-yl, 1,2,4-Triazol-1-yl, 1,2,3-Triazol-1-yl oder 1,2,3-Triazol-2-yl, wobei jede davon gegebenenfalls durch eine oder mehrere Gruppe(n) $R_2$ substituiert sein kann;

m Null, 1 oder 2 ist;

n eine ganze Zahl von 1 bis 5 ist;

q Null oder eine ganze Zahl von 1 bis 5 ist;

p Null oder 1 ist,

und landwirtschaftlich annehmbare Salze davon.

**2.** Verbindung nach Anspruch 1, in der

a) eine der als $(R_2)_n$ angegebenen Gruppen sich in ortho-Position an dem Phenylring befindet und/oder

b) sofern zwei als $(R_2)_n$ angegebene Gruppen vorliegen, diese sich in der 2- und 4-Position an dem Phenylring befinden und/oder

c) $R_1$ eine Alkylgruppe, die gegebenenfalls durch Halogen substituiert ist, oder eine Cycloalkylgruppe, die gegebenenfalls durch Alkyl oder Halogen substituiert ist, ist und/oder

d) $R_2$ ein Halogenatom oder eine Nitrogruppe oder $R_5$ oder $-S(O)_mR_5$ oder $-OR_5$ oder eine durch $-OR_5$ substituierte Alkylgruppe oder $-COOR_5$ ist und/oder

e) $R_5$ eine Alkylgruppe (mit 1 bis 3 Kohlenstoffatomen), gegebenenfalls durch Fluor oder Chlor substituiert, ist und/oder

f) $R_6$ Wasserstoff, Halogen, -OH, $-OR_5$, $-OCOR_8$ oder eine Alkylgruppe (mit 1 bis 4 Kohlenstoffatomen), gegebenenfalls durch ein oder mehrere Halogenatom(e) substituiert, ist und/oder

g) eine der Gruppen $R_6$ oder $R_7$ ein Wasserstoffatom ist und/oder

h) $R_7$ Wasserstoff oder eine Alkylgruppe mit 1 bis 4 Kohlenstoffatomen ist und/oder

i) $R_8$ eine Alkylgruppe mit 1 bis 4 Kohlenstoffatomen, gegebenenfalls substituiert durch ein oder mehrere Halogenatom(e), ist und/oder

j) n 2 oder 3 ist.

**3.** Verbindung nach Anspruch 1 oder 2, die

4-[Hydroxy-(2-nitro-4-trifluormethylphenyl)methyl]-5-cyclopropylisoxazol,
4-[Hydroxy-(2-nitro-4-trifluormethylphenyl)methyl]-5-methylisoxazol,
4-[Hydroxy-(2-chlor-3-ethoxy-4-methansulfonylphenyl)methyl]-5-cyclopropylisoxazol,
4-[Acetoxy-(2-nitro-4-trifluormethylphenyl)methyl]-5-cyclopropylisoxazol,
4-[Chlor-(2-nitro-4-trifluormethylphenyl)methyl]-5-cyclopropylisoxazol,
4-[Brom-(2-nitro-4-trifluormethylphenyl)methyl]-5-cyclopropylisoxazol,
4-[N,N-Dimethylamino-(2-nitro-4-trifluormethylphenyl)methyl]-5-cyclopropylisoxazol,
4-[Hydroxy-(2-chlor-4-methansulfonylphenyl)methyl]-5-cyclopropylisoxazol,
4-[Methoxy-(2-nitro-4-trifluormethylphenyl)methyl]-5-cyclopropylisoxazol,
4-[Hydroxy-(2-nitro-4-methylphenyl)methyl]-5-cyclopropylisoxazol,
4-[Hydroxy-(2-chlor-3-ethoxy-4-ethansulfonylphenyl)methyl]-5-methylisoxazol,
4-[Hydroxy-(2-chlor-3-ethoxy-4-ethansulfonylphenyl)methyl]-5-cyclopropylisoxazol,
4-[Acetoxy-(2-chlor-4-methansulfonylphenyl)methyl]-5-cyclopropylisoxazol,
4-[Methoxy-(2-chlor-4-methansulfonylphenyl)methyl]-5-cyclopropylisoxazol,
4-[Hydroxy-(2-chlor-4-fluorphenyl)methyl]-5-cyclopropylisoxazol,

4-[Hydroxy-(2-trifluormethyl-4-methansulfonylphenyl)methyl]-5-cyclopropylisoxazol,
4-[Acetoxy-(2-trifluormethyl-4-methansulfonylphenyl)methyl]-5-cyclopropylisoxazol,
4-[Methoxy-(2-trifluormethyl-4-methansulfonylphenyl)methyl]-5-cyclopropylisoxazol,
4-[Brom-(2-trifluormethyl-4-methansulfonylphenyl)methyl]-5-cyclopropylisoxazol,
4-[Chlor-(2-trifluormethyl-4-methansulfonylphenyl)methyl]-5-cyclopropylisoxazol,
4-[Trifluoracetoxy-(2-trifluormethyl-4-methansulfonylphenyl)methyl]-5-cyclopropylisoxazol,
4-[Hydroxy-(2-methansulfonylphenyl)methyl]-5-cyclopropylisoxazol,
4-[Hydroxy-(2,3-dichlor-4-methansulfonylphenyl)methyl]-5-(1-methylcyclopropyl)isoxazol,
4-[Hydroxy-(2-methansulfonyl-4-chlorphenyl)methyl]-5-cyclopropylisoxazol,
4-[Hydroxy-(2-brom-4-methansulfonylphenyl)methyl]-5-cyclopropylisoxazol,
4-[Hydroxy-(2-methansulfonyl-4-bromphenyl)methyl]-5-cyclopropylisoxazol,
4-[Hydroxy-(2-methyl-4-methansulfonylphenyl)methyl]-5-cyclopropylisoxazol,
4-[Hydroxy-(2-chlor-4-methanthiophenyl)methyl]-5-cyclopropylisoxazol,
4-[Hydroxy-(2-methansulfonyl-4-trifluormethylphenyl)methyl]-5-cyclopropylisoxazol,
4-[Hydroxy-(2-chlor-4-methansulfinylphenyl)methyl]-5-cyclopropylisoxazol,
4-(Hydroxy-(2-trifluormethyl-4-methanthiophenyl)methyl]-5-cyclopropylisoxazol,
4-[Hydroxy-(2-fluor-4-methansulfonylphenyl)methyl]-5-cyclopropylisoxazol,
4-[Hydroxy-(2-methansulfonyl-4-chlorphenyl)methyl]-5-isopropylisoxazol oder
4-[Hydroxy-(2-methansulfonyl-4-chlorphenyl)methyl]-5-(1-methylcyclopropyl)isoxazol ist.

**4.** Verfahren zur Herstellung einer Verbindung nach Anspruch 1, das umfaßt:

a) wenn $R_6$ eine OH-Gruppe ist und $R_7$ für ein Wasserstoffatom steht, die Reduktion einer Verbindung der allgemeinen Formel (II):

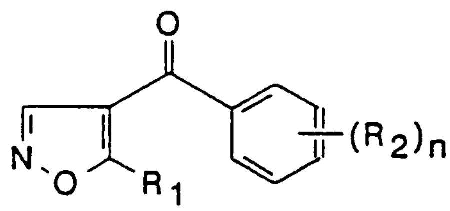

(II)

in der die anderen Symbole wie in Anspruch 1 definiert sind;

b) wenn $R_6$ eine OH-Gruppe und $R_7$ eine Gruppe $R_5$ ist, die Umsetzung einer Verbindung der allgemeinen Formel (II) mit einem geeigneten organometallischen Reaktionspartner in einem inerten Lösemittel;

c) wenn $R_6$ eine $-OCOR_8$-Gruppe oder eine -OCO-phenyl-$(R_2)_q$-Gruppe oder eine $-OCH_2$-phenyl-$(R_2)_q$-Gruppe oder eine -O-phenyl-$(R_2)_q$-Gruppe oder eine -OCO-Het1-Gruppe oder eine $-OCONR_3R_4$-Gruppe oder eine $-OSO_2R_8$-Gruppe oder eine $-OSO_2$-phenyl-$(R_2)_q$-Gruppe oder eine $-OSO_2NR_3R_4$-Gruppe und $R_7$ ein Wasserstoffatom oder eine Gruppe $R_5$ ist, die Umsetzung einer Verbindung der allgemeinen Formel (I), in der $R_6$ eine Hydroxygruppe und $R_7$ Wasserstoff oder eine Gruppe $R_5$ ist, mit der entsprechenden Halogenverbindung in Anwesenheit einer geeigneten Base;

d) wenn $R_6$ ein Chlor- oder Bromatom und $R_7$ ein Wasserstoffatom oder eine Gruppe $R_5$ ist, die Umsetzung einer Verbindung der allgemeinen Formel (I), in der $R_6$ eine Hydroxygruppe und $R_7$ ein Wasserstoffatom oder eine Gruppe $R_5$ ist, mit einem Halogenierungsmittel;

e) wenn $R_6$ ein Halogenatom oder eine Cyanogruppe und $R_7$ ein Wasserstoffatom oder eine Gruppe $R_5$ ist, zunächst die Hydroxylgruppe $R_6$ einer Verbindung der allgemeinen Formel (I), in der $R_6$ eine Hydroxylgruppe und $R_7$ ein Wasserstoffatom oder eine Gruppe $R_5$ ist, in eine Abgangsgruppe umzuwandeln, gefolgt von einer Umsetzung mit einem Alkalimetallhalogenid, einem Tetraalkylammoniumhalogenid oder einem Alkalimetallcyanid;

f) wenn $R_6$ eine $-OR_5$-Gruppe oder eine $-SR_5$-Gruppe oder eine Gruppe Het2 oder eine $-NR_3R_4$-Gruppe ist, die Substitution des Halogenatoms einer Verbindung der allgemeinen Formel (I), in der $R_6$ ein Halogenatom ist, durch ein geeignetes Nukleophil in einem inerten Lösemittel;

g) wenn $R_6$ eine -$SOR_5$-Gruppe oder eine -$SO_2R_5$-Gruppe ist, die Oxidation der entsprechenden Verbindung der allgemeinen Formel (I), in der $R_6$ eine -$SR_5$-Gruppe ist;

h) wenn $R_6$ eine -$OR_5$-Gruppe oder eine -$SR_5$-Gruppe und $R_7$ eine -$OR_5$-Gruppe oder eine -$SR_5$-Gruppe ist oder $R_6$ und $R_7$ für ein cyclisches Ketal oder cyclisches Thioketal stehen, die Behandlung von Verbindungen der allgemeinen Formel (II) mit dem geeigneten Alkohol oder Thiol in einem inerten Lösemittel in Anwesenheit eines Säurekatalysators;

i) wenn $R_6$ für eine -$COR_3$-Gruppe steht, wobei Wasserstoff für $R_3$ ausgeschlossen ist, die Umsetzung der entsprechenden Verbindung, in der $R_6$ für eine Cyanogruppe steht, mit einem organometallischen Reaktionspartner in einem inerten Lösemittel;

j) wenn $R_6$ für eine Aldehydgruppe -$COR_3$ steht, in der $R_3$ Wasserstoff darstellt, die Reduktion der entsprechenden Verbindung, in der $R_6$ für eine Cyanogruppe steht, unter Verwendung von Diisobutylaluminiumhydrid;

k) wenn $R_6$ für eine Gruppe -$NR_3COR_5$ steht, die Umsetzung der entsprechenden Verbindung, in der $R_6$ für eine -$NHR_3$-Gruppe steht, mit einer Acylverbindung der Formel $R_5CO$-X, in der X für eine Abgangsgruppe steht;

l) wenn $R_6$ für eine Amidgruppe, -$CONR_3R_4$, steht, die saure Hydrolyse der entsprechenden Verbindung, in der $R_6$ für eine Estergruppe -$CO_2R_5$ steht, gefolgt von einer Umwandlung der so erhaltenen Carbonsäure in das entsprechende Säurechlorid, gefolgt von einer Umwandlung in das Amid durch Behandlung mit einem Amin der Formel H-$NR_3R_4$;

m) wenn $R_6$ für eine Gruppe -$CSNR_3R_4$ steht, ein Umsetzen von Verbindungen, in denen $R_6$ für eine Gruppe -$CONR_3R_4$ steht, mit Lawesson's Reagens, [2,4-Bis(4-methoxyphenyl)-1,3-dithia-2,4-diphosphetan-2,4-disulfid];

n) wenn $R_6$ für eine Gruppe steht, ausgewählt aus $R_5$, einer Alkenylgruppe mit bis zu 6 Kohlenstoffatomen, die gegebenenfalls durch ein oder mehrere Halogenatom(e) substituiert sein kann, oder einer Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe -$COOR_5$ substituiert sein kann,

eine Verbindung der allgemeinen Formel (V)

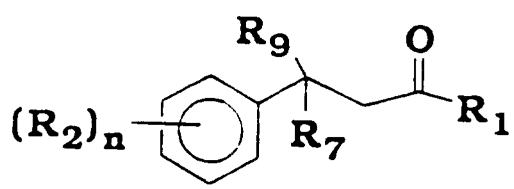

(V)

in der $R_9$ für eine Gruppe steht, ausgewählt aus $R_5$, einer Alkenylgruppe mit bis zu 6 Kohlenstoffatomen, die gegebenenfalls durch ein oder mehrere Halogenatom(e) substituiert sein kann, oder einer Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe -$COOR_5$ substituiert sein kann,

mit einem Trialkylorthoformiat in Anwesenheit eines Säurekatalysators unter Verwendung eines Salzes von Hydroxylamin umzusetzen;

o) wenn $R_6$ -$CO_2R_5$ ist, eine Hydrolyse der Cyanogruppe der entsprechenden Verbindung der allgemeinen Formel (I), in der $R_6$ eine Cyanogruppe ist, zu der entsprechenden Carbonsäure, die nachfolgend verestert wird;

und gegebenenfalls Umwandeln der so erhaltenen Verbindung der Formel (I) in ein landwirtschaftlich annehmbares Salz davon.

**5.** Herbizide Zusammensetzung, die als wirksamen Bestandteil eine herbizid wirksame Menge eines 4-Benzylisoxazols nach einem der Ansprüche 1 bis 3 oder ein landwirtschaftlich annehmbares Salz davon zusammen mit einem landwirtschaftlich annehmbaren Verdünnungsmittel oder Träger und/oder oberflächenaktiven Mittel umfaßt.

6. Herbizide Zusammensetzung nach Anspruch 5, die 0,05 bis 90 Gew.-% wirksamen Bestandteil umfaßt.

7. Herbizide Zusammensetzung nach Anspruch 5 oder 6, die in flüssiger Form vorliegt und 0,05 bis 25% oberflächenaktives Mittel enthält.

8. Herbizide Zusammensetzung nach einem der Ansprüche 5 bis 7 in Form eines wäßrigen Suspensionskonzentrats, eines benetzbaren Pulvers, eines wasserlöslichen oder in Wasser dispergierbaren Pulvers, eines flüssigen, wasserlöslichen Konzentrats, eines flüssigen, emulgierbaren Suspensionskonzentrats, eines Granulats oder eines emulgierbaren Konzentrats.

9. Verfahren zum Kontrollieren des Wachstums von Unkräutern an einem Standort, das umfaßt, an dem Standort eine herbizid wirksame Menge eines 4-Benzylisoxazolderivats nach einem der Ansprüche 1 bis 3 oder ein landwirtschaftlich annehmbares Salz davon auszubringen.

10. Verfahren nach Anspruch 9, bei dem der Standort eine Fläche ist, die zum Anbau von Feldfrüchten verwendet wird oder verwendet werden soll, und die Verbindung in einer Austragsmenge von 0,01 kg bis 4,0 kg pro Hektar angewendet wird.

11. Verfahren nach Anspruch 9, bei dem der Standort eine Fläche ist, die nicht zum Anbau von Feldfrüchten verwendet wird, und die Verbindung in einer Austragsmenge von 1,0 kg bis 20,0 kg pro Hektar angewendet wird.

12. Herstellungserzeugnis, umfassend mindestens ein Isoxazolderivat nach einem der Ansprüche 1 bis 3 oder eine herbizid wirksame Zusammensetzung nach einem der Ansprüche 5 bis 8 und vorzugsweise ein herbizid wirksames Konzentrat, das vor einer Verwendung verdünnt werden muß, wobei das Herstellungserzeugnis mindestens eines der Isoxazolderivate oder eine genannte herbizid wirksame Zusammensetzung in einem Behälter für das bzw. die vorstehend genannte(n) Derivat(e) und körperlich mit dem vorstehend genannten Behälter verbundene Anweisungen umfaßt, die die Art und Weise darlegen, wie das bzw. die vorstehend genannte(n) Derivat(e) oder die vorstehend genannte herbizid wirksame Zusammensetzung, das bzw. die darin enthalten ist bzw. sind, verwendet werden soll(en), um das Wachstum von Unkräutern zu kontrollieren.

**Patentansprüche für folgenden Vertragsstaat : ES**

1. Verfahren zur Herstellung eines 4-Benzylisoxazolderivats der allgemeinen Formel (I):

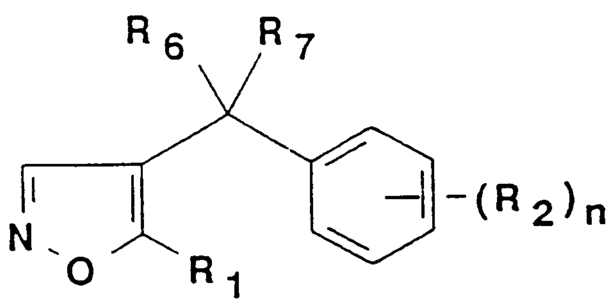

(I)

in der $R_1$ steht für:

eine gerad- oder verzweigtkettige Alkyl-, Alkenyl- oder Alkinylgruppe, die bis zu 6 Kohlenstoffatome enthält und die gegebenenfalls durch ein oder mehrere Halogenatom(e) substituiert sein kann, oder

eine Cycloalkylgruppe, die 3 bis 6 Kohlenstoffatome enthält und die gegebenenfalls durch eine oder mehrere Gruppe(n) $R_5$ oder ein oder mehrere Halogenatom(e) oder eine Gruppe $-COOR_5$ substituiert sein kann, oder

eine Cycloalkenylgruppe, die 5 oder 6 Kohlenstoffatome enthält und die gegebenenfalls durch eine oder mehrere Gruppe(n) $R_5$ oder ein oder mehrere Halogenatom(e) oder eine Gruppe $-COOR_5$ substituiert sein kann, oder

eine Aryl- oder Arylalkylgruppe der allgemeinen Formel $[(R_2)_q$phenyl$]-[C(R_3)(R_4)]_p-$ (wobei Aryl im allgemeinen C6-C10 und Arylalkyl im allgemeinen C7-C11 ist), oder
eine Estergruppe, $-COOR_5$, oder
eine Aldehyd- oder Acylgruppe, $-COR_3$, oder
eine Cyano- oder Nitrogruppe oder
eine Amingruppe, $-NR_3R_4$, oder
ein Halogenatom;

$R_2$ steht für:
eine Nitro- oder Cyanogruppe oder
ein Halogenatom oder
eine Gruppe $R_5$ oder
eine Sulfenyl-, Sulfinyl- oder Sulfonylgruppe, $-S(O)_mR_5$, oder
eine Sulfamoylgruppe, $-SO_2NR_3R_4$, oder
eine Estergruppe, $-COOR_5$, oder
eine Acyl- oder Aldehydgruppe, $-COR_3$, oder
eine Carbamoyl- oder Thiocarbamoylgruppe, $-CONR_3R_4$ oder $-CSNR_3R_4$, oder
eine Alkoxygruppe, $-OR_5$, oder
eine Alkylgruppe (mit 1 bis 3 Kohlenstoffatomen), die durch $-OR_5$ substituiert ist;

$R_3$ und $R_4$, die gleich oder unterschiedlich sein können, jeweils stehen für:
ein Wasserstoffatom oder eine gerad- oder verzweigtkettige Alkylgruppe, die bis zu 6 Kohlenstoffatome enthält und die gegebenenfalls durch ein oder mehrere Halogenatom(e) substituiert ist;

$R_5$ steht für:
eine gerad- oder verzweigtkettige Alkylgruppe, die bis zu 6 Kohlenstoffatome enthält und die gegebenenfalls durch ein oder mehrere Halogenatom(e) substituiert ist;

$R_6$ steht für:
ein Wasserstoffatom oder
eine Hydroxygruppe, OH, oder
ein Halogenatom oder
eine Gruppe $R_5$ oder
eine Alkenylgruppe mit bis zu 6 Kohlenstoffatomen, die gegebenenfalls durch ein oder mehrere Halogenatom(e) substituiert sein kann, oder
eine Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe $-COOR_5$ substituiert sein kann, oder
eine Sulfenyl-, Sulfinyl- oder Sulfonylgruppe, $-S(O)_mR_5$, oder
eine Estergruppe, $-COOR_5$, oder
eine Cyanogruppe oder
eine Acyl- oder Aldehydgruppe, $-COR_3$, oder
eine Carbamoyl- oder Thiocarbamoylgruppe, $-CONR_3R_4$ oder $-CSNR_3R_4$, oder
eine Alkoxygruppe, $-OR_5$, oder
eine Phenoxygruppe, $-O$-phenyl-$(R_2)_q$, oder
eine Benzyloxygruppe, $-OCH_2$-phenyl-$(R_2)_q$, oder
eine Acyloxygruppe, $-OCOR_8$, oder
eine Benzoyloxygruppe, $-OCO$-phenyl-$(R_2)_q$, oder
eine Gruppe -OCO-Het1 oder
eine Carbamoyloxygruppe, $-OCONR_3R_4$, oder
eine Alkylsulfonyloxygruppe, $-OSO_2R_8$, oder
eine Phenylsulfonyloxygruppe, $-OSO_2$-phenyl-$(R_2)_q$,
eine Sulfamoyloxygruppe, $-OSO_2NR_3R_4$, oder
eine Aminogruppe, $-NR_3R_4$, oder
eine Acylaminogruppe, $-NR_3COR_5$, oder
eine Gruppe Het2;

$R_7$ steht für:
ein Wasserstoffatom oder
eine Gruppe $R_5$;

oder $R_6$ und $R_7$ mit dem Kohlenstoffatom, an das sie gebunden sind, ein Ketal $((OR_5)_2)$ oder ein Thioketal $((SR_5)_2)$ oder ein cyclisches Ketal oder cyclisches Thioketal mit 5 oder 6 Atomen im Ring, gegebenenfalls substituiert durch eine oder mehrere Gruppe(n) $R_5$, bilden können;

$R_8$ steht für:

eine gerad- oder verzweigtkettige Alkyl- oder Alkenylgruppe mit bis zu 6 Kohlenstoffatomen, die gegebenenfalls durch ein oder mehrere Halogenatom(e) substituiert sein kann, oder

eine Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe $-COOR_5$ substituiert sein kann;

wobei Het1 für einen Heterocyclus mit 5 oder 6 Atomen im Ring steht, von denen eines oder mehrere ein Heteroatom, ausgewählt aus Stickstoff, Schwefel und Sauerstoff, ist bzw. sind, wobei die Ringkohlenstoffatome gegebenenfalls durch eine Gruppe $R_2$ substituiert sind;

wobei Het2 für eine heterocyclische Gruppe steht, ausgewählt aus Pyrrol-1-yl, Pyrazol-1-yl, Imidazol-1-yl, 1,2,4-Triazol-4-yl, 1,2,4-Triazol-1-yl, 1,2,3-Triazol-1-yl oder 1,2,3-Triazol-2-yl, wobei jede davon gegebenenfalls durch eine oder mehrere Gruppe(n) $R_2$ substituiert sein kann;

m Null, 1 oder 2 ist;

n eine ganze Zahl von 1 bis 5 ist;

q Null oder eine ganze Zahl von 1 bis 5 ist;

p Null oder 1 ist,

oder ein landwirtschaftlich annehmbares Salz davon, wobei das Verfahren umfaßt

a) wenn $R_6$ eine OH-Gruppe ist und $R_7$ für ein Wasserstoffatom steht, die Reduktion einer Verbindung der allgemeinen Formel (II):

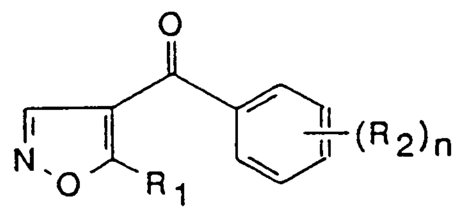

(II)

in der die anderen Symbole wie vorstehend definiert sind;

b) wenn $R_6$ eine OH-Gruppe und $R_7$ eine Gruppe $R_5$ ist, die Umsetzung einer Verbindung der allgemeinen Formel (II) mit einem geeigneten organometallischen Reaktionspartner in einem inerten Lösemittel;

c) wenn $R_6$ eine $-OCOR_8$-Gruppe oder eine $-OCO\text{-phenyl-}(R_2)_q$-Gruppe oder eine $-OCH_2\text{-phenyl-}(R_2)_q$-Gruppe oder eine $-O\text{-phenyl-}(R_2)_q$-Gruppe oder eine -OCO-Het1-Gruppe oder eine $-OCONR_3R_4$-Gruppe oder eine $-OSO_2R_8$-Gruppe oder eine $-OSO_2\text{-phenyl-}(R_2)_q$-Gruppe oder eine $-OSO_2NR_3R_4$-Gruppe und $R_7$ ein Wasserstoffatom oder eine Gruppe $R_5$ ist, die Umsetzung einer Verbindung der allgemeinen Formel (I), in der $R_6$ eine Hydroxygruppe und $R_7$ Wasserstoff oder eine Gruppe $R_5$ ist, mit der entsprechenden Halogenverbindung in Anwesenheit einer geeigneten Base;

d) wenn $R_6$ ein Chlor- oder Bromatom und $R_7$ ein Wasserstoffatom oder eine Gruppe $R_5$ ist, die Umsetzung einer Verbindung der allgemeinen Formel (I), in der $R_6$ eine Hydroxygruppe und $R_7$ für ein Wasserstoffatom oder eine Gruppe $R_5$ steht, mit einem Halogenierungsmittel;

e) wenn $R_6$ ein Halogenatom oder eine Cyanogruppe und $R_7$ ein Wasserstoffatom oder eine Gruppe $R_5$ ist, zunächst die Hydroxylgruppe $R_6$ einer Verbindung der allgemeinen Formel (I), in der $R_6$ eine Hydroxylgruppe und $R_7$ ein Wasserstoffatom oder eine Gruppe $R_5$ ist, in eine Abgangsgruppe umzuwandeln, gefolgt von einer Umsetzung mit einem Alkalimetallhalogenid, einem Tetraalkylammoniumhalogenid oder einem Alkalimetallcyanid;

f) wenn $R_6$ eine $-OR_5$-Gruppe oder eine $-SR_5$-Gruppe oder eine Gruppe Het2 oder eine $-NR_3R_4$-Gruppe ist, die Substitution des Halogenatoms einer Verbindung der allgemeinen Formel (I), in der $R_6$ ein Halogenatom ist, durch ein geeignetes Nukleophil in einem inerten Lösemittel;

g) wenn $R_6$ eine $-SOR_5$-Gruppe oder eine $-SO_2R_5$-Gruppe ist, die Oxidation der entsprechenden Verbindung der allgemeinen Formel (I) , in der $R_6$ eine $-SR_5$-Gruppe ist;

h) wenn $R_6$ eine $-OR_5$ -Gruppe oder eine $-SR_5$-Gruppe und $R_7$ eine $-OR_5$-Gruppe oder eine $-SR_5$-Gruppe ist oder $R_6$ und $R_7$ für ein cyclisches Ketal oder cyclisches Thioketal stehen, die Behandlung von Verbindungen der allgemeinen Formel (II) mit dem geeigneten Alkohol oder Thiol in einem inerten Lösemittel in Anwesenheit eines Säurekatalysators;

i) wenn $R_6$ für eine $-COR_3$-Gruppe steht, wobei Wasserstoff für $R_3$ ausgeschlossen ist, die Umsetzung der entsprechenden Verbindung, in der $R_6$ für eine Cyanogruppe steht, mit einem organometallischen Reaktionspartner in einem inerten Lösemittel;

j) wenn $R_6$ für eine Aldehydgruppe $-COR_3$ steht, in der $R_3$ Wasserstoff darstellt, die Reduktion der entsprechenden Verbindung, in der $R_6$ für eine Cyanogruppe steht, unter Verwendung von Diisobutylaluminiumhydrid;

k) wenn $R_6$ für eine Gruppe $-NR_3COR_5$ steht, die Umsetzung der entsprechenden Verbindung, in der $R_6$ für eine $-NHR_3$-Gruppe steht, mit einer Acylverbindung der Formel $R_5CO-X$, in der X für eine Abgangsgruppe steht;

l) wenn $R_6$ für eine Amidgruppe, $-CONR_3R_4$, steht, die saure Hydrolyse der entsprechenden Verbindung, in der $R_6$ für eine Estergruppe $-CO_2R_5$ steht, gefolgt von einer Umwandlung der so erhaltenen Carbonsäure in das entsprechende Säurechlorid, gefolgt von einer Umwandlung in das Amid durch Behandlung mit einem Amin der Formel $H-NR_3R_4$;

m) wenn $R_6$ für eine Gruppe $-CSNR_3R_4$ steht, ein Umsetzen von Verbindungen, in denen $R_6$ für eine Gruppe $-CONR_3R_4$ steht, mit Lawesson's Reagens, [2,4-Bis(4-methoxyphenyl)-1,3-dithia-2,4-diphosphetan-2,4-disulfid];

n) wenn $R_6$ für eine Gruppe steht, ausgewählt aus $R_5$, einer Alkenylgruppe mit bis zu 6 Kohlenstoffatomen, die gegebenenfalls durch ein oder mehrere Halogenatom(e) substituiert sein kann, oder einer Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe $-COOR_5$ substituiert sein kann,

eine Verbindung der allgemeinen Formel (V)

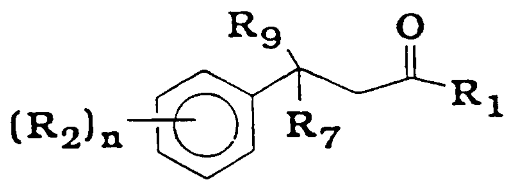

(V)

in der $R_9$ für eine Gruppe steht, ausgewählt aus $R_5$, einer Alkenylgruppe mit bis zu 6 Kohlenstoffatomen, die gegebenenfalls durch ein oder mehrere Halogenatom(e) substituiert sein kann, oder einer Cycloalkylgruppe mit 3 bis 6 Kohlenstoffatomen, die gegebenenfalls durch eine oder mehrere Gruppe(n) $R_5$ oder ein oder mehrere Halogenatom(e) oder eine Gruppe $-COOR_5$ substituiert sein kann,

mit einem Trialkylorthoformiat in Anwesenheit eines Säurekatalysators unter Verwendung eines Salzes von Hydroxylamin umzusetzen;

o) wenn $R_6$ $-CO_2R_5$ ist, die Cyanogruppe der entsprechenden Verbindung der allgemeinen Formel (I), in der $R_6$ eine Cyanogruppe ist, zu der entsprechenden Carbonsäure, die nachfolgend verestert wird, zu hydrolysieren;

und gegebenenfalls Umwandeln der so erhaltenen Verbindung der Formel (I) in ein landwirtschaftlich annehmbares Salz davon.

**2.** Verfahren nach Anspruch 1 zur Herstellung einer Verbindung der allgemeinen Formel (I), in der:

a) eine der durch $(R_2)_n$ dargestellten Gruppen sich in ortho-Position an dem Phenylring befindet und/oder

b) sofern zwei durch $(R_2)_n$ dargestellte Gruppen vorliegen, diese sich in der 2- und 4-Position an dem Phenylring befinden und/oder

c) $R_1$ eine Alkylgruppe, die gegebenenfalls durch Halogen substituiert ist, oder eine Cycloalkylgruppe, die gegebenenfalls durch Alkyl oder Halogen substituiert ist, ist und/oder

d) $R_2$ ein Halogenatom oder eine Nitrogruppe oder $R_5$ oder $-S(O)_mR_5$ oder $-OR_5$ oder eine durch $-OR_5$ substituierte Alkylgruppe oder $-COOR_5$ ist und/oder

e) $R_5$ eine Alkylgruppe (mit 1 bis 3 Kohlenstoffatomen), gegebenenfalls durch Fluor oder Chlor substituiert, ist und/oder

f) $R_6$ Wasserstoff, Halogen, -OH, $-OR_5$, $-OCOR_8$ oder eine Alkylgruppe (mit 1 bis 4 Kohlenstoffatomen), gegebenenfalls durch ein oder mehrere Halogenatom(e) substituiert, ist und/oder

g) eine der Gruppen $R_6$ oder $R_7$ ein Wasserstoffatom ist und/oder

h) $R_7$ Wasserstoff oder eine Alkylgruppe mit 1 bis 4 Kohlenstoffatomen ist und/oder

i) $R_8$ eine Alkylgruppe mit 1 bis 4 Kohlenstoffatomen, die gegebenenfalls durch ein oder mehrere Halogenatom(e) substituiert ist, ist und/oder

j) n 2 oder 3 ist.

**3.** Verfahren nach Anspruch 1 oder 2 zur Herstellung einer Verbindung der allgemeinen Formel (I), die
4-[Hydroxy-(2-nitro-4-trifluormethylphenyl)methyl]-5-cyclopropylisoxazol,
4-[Hydroxy-(2-nitro-4-trifluormethylphenyl)methyl]-5-methylisoxazol,
4-[Hydroxy-(2-chlor-3-ethoxy-4-methansulfonylphenyl)methyl]-5-cyclopropylisoxazol,
4-[Acetoxy-(2-nitro-4-trifluormethylphenyl)methyl]-5-cyclopropylisoxazol,
4-[Chlor-(2-nitro-4-trifluormethylphenyl)methyl]-5-cyclopropylisoxazol,
4-[Brom-(2-nitro-4-trifluormethylphenyl)methyl]-5-cyclopropylisoxazol,
4-[N,N-Dimethylamino-(2-nitro-4-trifluormethylphenyl)methyl]-5-cyclopropylisoxazol,
4-[Hydroxy-(2-chlor-4-methansulfonylphenyl)methyl]-5-cyclopropylisoxazol,
4-[Methoxy-(2-nitro-4-trifluormethylphenyl)methyl]-5-cyclopropylisoxazol,
4-[Hydroxy-(2-nitro-4-methylphenyl)methyl]-5-cyclopropylisoxazol,
4-[Hydroxy-(2-chlor-3-ethoxy-4-ethansulfonylphenyl)methyl]-5-methylisoxazol,
4-[Hydroxy-(2-chlor-3-ethoxy-4-ethansulfonylphenyl)methyl]-5-cyclopropylisoxazol,
4-[Acetoxy-(2-chlor-4-methansulfonylphenyl)methyl]-5-cyclopropylisoxazol,
4-[Methoxy-(2-chlor-4-methansulfonylphenyl)methyl]-5-cyclopropylisoxazol,
4-[Hydroxy-(2-chlor-4-fluorphenyl)methyl]-5-cyclopropylisoxazol,
4-[Hydroxy-(2-trifluormethyl-4-methansulfonylphenyl)methyl]-5-cyclopropylisoxazol,
4-[Acetoxy-(2-trifluormethyl-4-methansulfonylphenyl)methyl]-5-cyclopropylisoxazol,
4-[Methoxy-(2-trifluormethyl-4-methansulfonylphenyl)methyl]-5-cyclopropylisoxazol,
4-[Brom-(2-trifluormethyl-4-methansulfonylphenyl)methyl]-5-cyclopropylisoxazol,
4-[Chlor-(2-trifluormethyl-4-methansulfonylphenyl)methyl]-5-cyclopropylisoxazol,
4-[Trifluoracetoxy-(2-trifluormethyl-4-methansulfonylphenyl)methyl]-5-cyclopropylisoxazol,
4-[Hydroxy-(2-methansulfonylphenyl)methyl]-5-cyclopropylisoxazol,
4-[Hydroxy-(2,3-dichlor-4-methansulfonylphenyl)methyl]-5-(1-methylcyclopropyl)isoxazol,
4-[Hydroxy-(2-methansulfonyl-4-chlorphenyl)methyl]-5-cyclopropylisoxazol,
4-[Hydroxy-(2-brom-4-methansulfonylphenyl)methyl]-5-cyclopropylisoxazol,
4-[Hydroxy-(2-methansulfonyl-4-bromphenyl)methyl]-5-cyclopropylisoxazol,
4-[Hydroxy-(2-methyl-4-methansulfonylphenyl)methyl]-5-cyclopropylisoxazol,
4-[Hydroxy-(2-chlor-4-methanthiophenyl)methyl]-5-cyclopropylisoxazol,
4-[Hydroxy-(2-methansulfonyl-4-trifluormethylphenyl)methyl]-5-cyclopropylisoxazol,
4-[Hydroxy-(2-chlor-4-methansulfinylphenyl)methyl]-5-cyclopropylisoxazol,
4-[Hydroxy-(2-trifluormethyl-4-methanthiophenyl)methyl]-5-cyclopropylisoxazol,
4-[Hydroxy-(2-fluor-4-methansulfonylphenyl)methyl]-5-cyclopropylisoxazol,
4-[Hydroxy-(2-methansulfonyl-4-chlorphenyl)methyl]-5-isopropylisoxazol oder
4-[Hydroxy-(2-methansulfonyl-4-chlorphenyl)methyl]-5-(1-methylcyclopropyl)isoxazol ist.

**4.** Verfahren zur Herstellung einer herbizid wirksamen Zusammensetzung, das umfaßt als wirksamen Bestandteil eine herbizid wirksame Menge eines 4-Benzylisoxazols, wie in einem der Ansprüche 1 bis 3 definiert, oder ein landwirtschaftlich annehmbares Salz davon mit einem landwirtschaftlich annehmbaren Verdünnungsmittel oder Träger und/oder oberflächenaktiven Mittel zu formulieren.

**5.** Verfahren nach Anspruch 4,
wobei die herbizid wirksame Zusammensetzung 0,05 bis 90 Gew.-% wirksamen Bestandteil umfaßt.

**6.** Verfahren nach Anspruch 4 oder 5,
wobei die herbizid wirksame Zusammensetzung in flüssiger Form vorliegt und 0,05 bis 25% oberflächenaktives Mittel enthält.

7. Verfahren nach einem der Ansprüche 4 bis 6,
wobei die herbizid wirksame Zusammensetzung in Form eines wäßrigen Suspensionskonzentrats, eines benetzbaren Pulvers, eines wasserlöslichen oder in Wasser dispergierbaren Pulvers, eines flüssigen, wasserlöslichen Konzentrats, eines flüssigen, emulgierbaren Suspensionskonzentrats, eines Granulats oder eines emulgierbaren Konzentrats vorliegt.

8. Verfahren zum Kontrollieren des Wachstums von Unkräutern an einem Standort, das umfaßt, an dem Standort eine herbizid wirksame Menge eines 4-Benzylisoxazolderivats, wie in einem der Ansprüche 1 bis 3 definiert, oder ein landwirtschaftlich annehmbares Salz davon auszubringen.

9. Verfahren nach Anspruch 8, bei dem der Standort eine Fläche ist, die zum Anbau von Feldfrüchten verwendet wird oder verwendet werden soll, und die Verbindung in einer Austragsmenge von 0,01 kg bis 4,0 kg pro Hektar angewendet wird.

10. Verfahren nach Anspruch 8, bei dem der Standort eine Fläche ist, die nicht zum Anbau von Feldfrüchten verwendet wird, und die Verbindung in einer Austragsmenge von 1,0 kg bis 20,0 kg pro Hektar angewendet wird.

11. Verfahren zum Herstellen eines Herstellungserzeugnisses, umfassend mindestens ein Isoxazolderivat, wie in einem der Ansprüche 1 bis 3 definiert, oder eine herbizid wirksame Zusammensetzung, wie in einem der Ansprüche 4 bis 7 definiert, und vorzugsweise ein herbizid wirksames Konzentrat, das vor einer Verwendung verdünnt werden muß,
wobei das Verfahren umfaßt, das bzw. die Isoxazolderivat(e) oder die herbizid wirksame Zusammensetzung in einen Behälter abzufüllen und in körperlicher Verbindung mit dem vorstehend genannten Behälter Anweisungen anzuordnen, die die Art und Weise darlegen, wie das bzw. die vorstehend genannte(n) Derivat(e) oder die vorstehend genannte herbizid wirksame Zusammensetzung, das bzw. die darin enthalten ist bzw. sind, verwendet werden soll(en), um das Wachstum von Unkräutern zu kontrollieren.

**Revendications**
**Revendications pour les Etats contractants suivants : AT, BE, CH, LI, DE, DK, FR, GB, GR, IT, LU, NL, SE**

1. Dérivés de 4-benzylisoxazole de formule générale (I) :

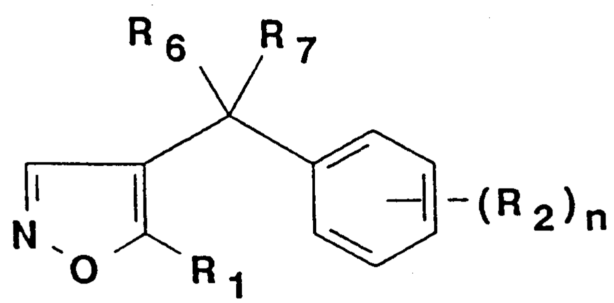

(I)

dans laquelle $R_1$ représente :

un groupe alkyle, alcényle ou alcynyle à chaîne droite ou à chaîne ramifiée contenant jusqu'à 6 atomes de carbone, qui peut facultativement être substitué par un ou plusieurs atomes d'halogènes ; ou bien

un groupe cycloalkyle contenant 3 à 6 atomes de carbone, qui peut être facultativement substitué par un ou plusieurs groupes $R_5$ et un ou plusieurs atomes d'halogènes ou un groupe $-COOR_5$ ; ou bien

un groupe cycloalcényle contenant 5 ou 6 atomes de carbone, qui peut être facultativement substitué par un ou plusieurs groupes $R_5$ ou par un ou plusieurs atomes d'halogènes ou un groupe $COOR_5$ ; ou bien

un groupe aryle ou aralkyle de formule générale $[(R_2)_q$phényl$]-[C(R_3)(R_4)]_p$-(le groupe aryle est généralement en $C_6$ à $C_{10}$ et le groupe aralkyle est généralement en $C_7$ à $C_{11}$) ; ou bien
un groupe ester, $COOR_5$ ; ou bien
un groupe aldéhyde ou acyle, $COR_3$ ; ou bien
un groupe cyano ou nitro ; ou bien
un groupe amino, $-NR_3R_4$ ; ou bien
un atome d'halogène ;

$R_2$ représente :
un groupe nitro ou cyano ; ou
un atome d'halogène ; ou
un groupe $R_5$ ; ou
un groupe sulfényle, sulfinyle ou sulfonyle, $-S(O)_mR_5$ ; ou
un groupe sulfamoyle, $-SO_2NR_3R_4$ ; ou
un groupe ester, $-COOR_5$ ; ou
un groupe acyle ou aldéhyde, $-COR_3$ ; ou
un groupe carbamoyle ou thiocarbamoyle $-CONR_3R_4$ ou $-CSNR_3R_4$ ; ou
un groupe alkoxy, $-OR_5$ ; ou
un groupe alkyle (ayant 1 à 3 atomes de carbone substitué par $-OR_5$ ;
$R_3$ et $R_4$, qui peuvent être identiques ou différents, représentent chacun :
un atome d'hydrogène ou un groupe alkyle à chaîne droite ou à chaîne ramifiée contenant jusqu'à 6 atomes de carbone, qui est facultativement substitué par un ou plusieurs atomes d'halogènes ;

$R_5$ représente :
un groupe alkyle à chaîne droite ou à chaîne ramifiée contenant jusqu'à 6 atomes de carbone, qui est facultativement substitué par un ou plusieurs atomes d'halogènes ;

$R_6$ représente :
un atome d'hydrogène ; ou
un groupe hydroxy, OH ; ou
un atome d'halogène ; ou
un groupe $R_5$ ; ou
un groupe alcényle contenant jusqu'à 6 atomes de carbone, qui peut être facultativement substitué par un ou plusieurs atomes d'halogènes ; ou
un groupe cycloalkyle contenant 3 à 6 atomes de carbone qui peut être facultativement substitué par un ou plusieurs groupes $R_5$ ou par un ou plusieurs atomes d'halogènes ou un groupe $-COOR_5$ ; ou bien
un groupe sulfényle, sulfinyle ou sulfonyle, $-S(O)_mR_5$ ;
un groupe ester, $-COOR_5$ ; ou
un groupe cyano ; ou
un groupe acyle ou aldéhyde, $-COR_3$ ; ou
un groupe carbamoyle ou thiocarbamoyle, $-CONR_3R_4$ ou $-CSNR_3R_4$ ;

ou
un groupe alkoxy, $-OR_5$ ; ou
un groupe phénoxy, -O-phényl-$(R_2)_q$ ; ou
un groupe benzyloxy, $-OCH_2$-phényl-$(R_2)_q$ ; ou
un groupe acyloxy, $-OCOR_8$ ; ou
un groupe benzoyloxy, -OCO-phényl-$(R_2)_q$ ; ou
un groupe -OCO-Het1 ; ou
un groupe carbamoyloxy, $-OCONR_3R_4$ ; ou
un groupe alkylsulfonyloxy, $-OSO_2R_8$ ; ou
un groupe phénylsulfonyloxy, $-OSO_2$-phényl-$(R_2)_q$,
un groupe sulfamoyloxy, $-OSO_2NR_3R_4$ ; ou
un groupe amino, $-NR_3R_4$ ; ou
un groupe acylamino, $-NR_3COR_5$ ; ou
un groupe Het2 ;

$R_7$ représente :
un atome d'hydrogène ; ou
un groupe $R_5$ ;

ou bien $R_6$ et $R_7$ peuvent former avec l'atome de carbone auquel ils sont attachés, un cétal $((OR_5)_2)$, ou un thiocétal $((SR_5)_2)$ ou un cétal cyclique ou un thiocétal cyclique contenant 5 ou 6 atomes de

carbone dans le noyau, facultativement substitué par un ou plusieurs groupes $R_5$ ;
$R_8$ représente :

un groupe alkyle ou alcényle à chaîne droite ou à chaîne ramifiée contenant jusqu'à 6 atomes de carbone, qui peut facultativement être substitué par un ou plusieurs atomes d'halogènes ; ou

un groupe cycloalkyle contenant 3 à 6 atomes de carbone qui peut facultativement être substitué par un ou plusieurs groupes $R_5$ ou par un ou plusieurs atomes d'halogènes ou un groupe $-COOR_5$ ;

Het1 représente un hétérocycle contenant 5 ou 6 atomes dans le noyau, un ou plusieurs de ces atomes consistant en un hétéroatome choisi entre azote, soufre et oxygène, les atomes de carbone du noyau étant facultativement substitués par un groupe $R_2$ ;

Het2 représente un groupe hétérocyclique choisi entre : pyrrol-1-yle, pyrazole-1-yle, imidazole-1-yle, 1,2,4-triazole-4-yle, 1,2,4-triazoyl-1-yle, 1,2,3-triazole-1-yle ou 1,2,3-triazole-2-yle ; chacun d'eux pouvant facultativement être substitué par un ou plusieurs groupes $R_2$ ;

m a la valeur zéro, 1 ou 2 ;
n représente un nombre entier de 1 à 5 ;
q est égal à zéro ou à un nombre entier de 1 à 5 ;
p a la valeur zéro ou 1 ;
et leurs sels acceptables en agriculture.

**2.** Composé suivant la revendication 1, dans lequel :

a) l'un des groupes représentés par $(R_2)_n$ est en position ortho sur le noyau phényle ; et/ou
b) lorsqu'il y a deux groupes représentés par $(R_2)_n$, ils sont en positions 2 et 4 sur le noyau phényle ; et/ou
c) $R_1$ est un groupe alkyle facultativement substitué par un halogène ; ou un groupe cycloalkyle facultativement substitué par un radical alkyle ou un halogène ; et/ou
d) $R_2$ est un halogène ; ou un groupe nitro ; ou un groupe $R_5$ ; ou un groupe $-S(O)_mR_5$ ; ou $-OR_5$ ; ou un groupe alkyle substitué par $-OR_5$ ; ou un groupe $-COOR_5$ ; et/ou
e) $R_5$ est un groupe alkyle (ayant 1 à 3 atomes de carbone) facultativement substitué par du fluor ou du chlore ; et/ou
f) $R_6$ est de l'hydrogène, un halogène, un groupe -OH, $-OR_5$, $-OCOR_8$ ou un groupe alkyle (ayant un à quatre atomes de carbone) facultativement substitués par un ou plusieurs atomes d'halogènes ; et/ou
g) l'un des groupes $R_6$ et $R_7$ est un atome d'hydrogène ; et/ou
h) $R_7$ est un atome d'hydrogène ou un groupe alkyle contenant un à quatre atomes de carbone ; et/ou
i) $R_8$ est un groupe alkyle contenant un à quatre atomes de carbone facultativement substitués par un ou plusieurs atomes d'halogènes ; et/ou
j) n a la valeur 2 ou 3.

**3.** Composé suivant la revendication 1 ou 2, qui est :

le 4-[hydroxy-(2-nitro-4-trifluorométhylphényl)méthyl]-5-cyclopropylisoxazole ;
le 4-[hydroxy-(2-nitro-4-trifluorométhylphényl)méthyl]-5-méthylisoxazole ;
le 4-[hydroxy-(2-chloro-3-éthoxy-4-méthanesulfonylphényl)méthyl]-5-cyclopropylisoxazole ;
le 4-[acétoxy-(2-nitro-4-trifluorométhylphényl)méthyl]-5-cyclopropylisoxazole ;
le 4-[chloro-(2-nitro-4-trifluorométhylphényl)méthyl]-5-cyclopropylisoxazole ;
le 4-[bromo-(2-nitro-4-trifluorométhylphényl)méthyl]-5-cyclopropylisoxazole ;
le 4-[N,N-diméthylamino-(2-nitro-4-trifluorométhylphényl)méthyl]-5-cyclopropylisoxazole ;
le 4-[hydroxy-(2-chloro-4-méthanesulfonylphényl)méthyl]-5-cyclopropylisoxazole ;
le 4-[méthoxy-(2-nitro-4-trifluorométhylphényl)méthyl]-5-cyclopropylisoxazole ;
le 4-[hydroxy-(2-nitro-4-méthylphényl)méthyl]-5-cyclopropylisoxazole ;
le 4-[hydroxy-(2-chloro-3-éthoxy-4-éthanesulfonylphényl)méthyl]-5-méthylisoxazole ;
le 4-[hydroxy-(2-chloro-3-éthoxy-4-éthanesulfonylphényl)méthyl]-5-cyclopropylisoxazole ;
le 4-[acétoxy-(2-chloro-4-méthanesulfonylphényl)méthyl]-5-cyclopropylisoxazole ;
le 4-[méthoxy-(2-chloro-4-méthanesulfonylphényl)méthyl]-5-cyclopropylisoxazole ;
le 4-[hydroxy-(2-chloro-4-fluorophényl)méthyl]-5-cyclopropylisoxazole ;
le 4-[hydroxy-(2-trifluorométhyl-4-méthanesulfonylphényl)méthyl]-5-cyclopropylisoxazole ;
le 4-[acétoxy-(2-trifluorométhyl-4-méthanesulfonylphényl)méthyl]-5-cyclopropylisoxazole ;
le 4-[méthoxy-(2-trifluorométhyl-4-méthanesulfonylphényl)méthyl]-5-cyclopropylisoxazole ;
le 4-[bromo-(2-trifluorométhyl-4-méthanesulfonylphényl)méthyl]-5-cyclopropylisoxazole ;

le 4-[chloro-(2-trifluorométhyl-4-méthanesulfonylphényl)méthyl]-5-cyclopropylisoxazole ;
le 4-[trifluoroacétoxy-(2-trifluorométhyl-4-méthanesulfonylphényl)-méthyl]-5-cyclopropylisoxazole ;
le 4-[hydroxy-(2-méthanesulfonylphényl)méthyl]-5-cyclopropylisoxazole ;
le 4-[hydroxy(2,3-dichloro-4-méthanesulfonylphényl)méthyl]-5-(1-méthylcyclopropyl)isoxazole ;
le 4-[hydroxy-(2-méthanesulfonyl-4-chlorophényl)méthyl]-5-cyclopropylisoxazole ;
le4-[hydroxy-(2-bromo-4-méthanesulfonylphényl)méthyl]-5-cyclopropylisoxazole ;
le 4-[hydroxy-(2-méthanesulfonyl-4-bromophényl)méthyl]-5-cyclopropylisoxazole ;
le 4-[hydroxy-(2-méthyl-4-méthanesulfonylphényl)méthyl]-5-cyclopropylisoxazole ;
le 4-[hydroxy-(2-chloro-4-méthanethiophényl)méthyl]-5-cyclopropylisoxazole ;
le 4-[hydroxy-(2-méthanesulfonyl-4-trifluorométhylphényl)méthyl]-5-cyclopropylisoxazole ;
le 4-[hydroxy-(2-chloro-4-méthanesulfinylphényl)méthyl]-5-cyclopropylisoxazole ;
le 4-[hydroxy-(2-trifluorométhyl-4-méthanethiophényl)méthyl]-5-cyclopropylisoxazole ;
le 4-[hydroxy-(2-fluoro-4-méthanesulfonylphényl)méthyl]-5-cyclopropylisoxazole ;
le 4-[hydroxy-(2-méthanesulfonyl-4-chlorophényl)méthyl]-5-isopropylisoxazole ; ou
le 4-[hydroxy-(2-méthanesulfonyl-4-chlorophényl)méthyl]-5-(1-méthylcyclopropyl)isoxazole.

**4.** Procédé de production d'un composé suivant la revendication 1, qui comprend :

a) lorsque $R_6$ est un groupe OH et $R_7$ représente l'atome d'hydrogène, la réduction d'un composé de formule générale (II) :

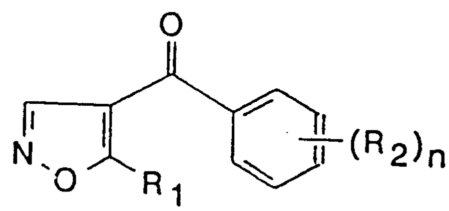

(II)

dans laquelle les autres symboles sont tels que définis dans la revendication 1 ;

b) lorsque $R_6$ est un groupe -OH et $R_7$ est un groupe $R_5$, la réaction d'un composé de formule générale (II) avec un réactif organométallique approprié dans un solvant inerte ;

c) lorsque $R_6$ est un groupe $-OCOR_8$ ou un groupe -OCO-phényl-$(R_2)_q$ ou un groupe $-OCH_2$-phényl-$(R_2)_q$ ou un groupe -O-phényl-$(R_2)_q$ ou un groupe -OCO-Het1 ou un groupe $-OCONR_3R_4$ ou un groupe $-OSO_2R_8$ ou un groupe $-OSO_2$-phényl-$(R_2)_q$ ou un groupe $-OSO_2NR_3R_4$ et $R_7$ est un atome d'hydrogène ou un groupe $R_5$,

la réaction d'un composé de formule générale (I) dans laquelle $R_6$ est un groupe hydroxy et $R_7$ est l'hydrogène ou un groupe $R_5$, avec le composé halogéné correspondant en présence d'une base convenable ;

d) lorsque $R_6$ est un atome de chlore ou de brome et $R_7$ est un atome d'hydrogène ou un groupe $R_5$, la réaction d'un composé de formule générale (I) dans laquelle $R_6$ est un groupe hydroxy et $R_7$ est un atome d'hydrogène ou un groupe $R_5$ avec un agent d'halogénation ;

e) lorsque $R_6$ est un atome d'halogène ou un groupe cyano et $R_7$ est un atome d'hydrogène ou un groupe $R_5$, tout d'abord la transformation du groupe hydroxyle $R_6$ d'un composé de formule générale (I) dans laquelle $R_6$ est un groupe hydroxyle et $R_7$ est un atome d'hydrogène ou un groupe $R_5$ en un groupe partant, suivie de la réaction avec un halogénure de métal alcalin, un halogénure de tétraalkylammonium ou un cyanure de métal alcalin ;

f) lorsque $R_6$ est un groupe $-OR_5$ ou un groupe $-SR_5$ ou un groupe Het2 ou un groupe $-NR_3R_4$, le remplacement de l'atome d'halogène d'un composé de formule générale (I) dans laquelle $R_6$ est un atome d'halogène par un groupe nucléophile convenable dans un solvant inerte ;

g) lorsque $R_6$ est un groupe $-SOR_5$ ou un groupe $-SO_2R_5$, l'oxydation du composé correspondant de formule générale (I) dans laquelle $R_6$ est un groupe $-SR_5$ ;

h) lorsque $R_6$ est un groupe $-OR_5$ ou un groupe $-SR_5$ et $R_7$ est un groupe $-OR_5$ ou un groupe $-SR_5$, ou bien $R_6$ et $R_7$ représentent un cétal cyclique ou un thiocétal cyclique, le traitement de composés de formule générale (II) avec l'alcool ou le thio approprié dans un solvant inerte en présence d'un

catalyseur acide ;

i) lorsque $R_6$ représente un groupe $-COR_3$ dans lequel $R_3$ exclue l'hydrogène, la réaction du composé correspondant dans lequel $R_6$ représente un groupe cyano avec un réactif organométallique dans un solvant inerte ;

j) lorsque $R_6$ représente un groupe aldéhyde $-COR_3$ dans lequel $R_3$ est l'hydrogène, la réduction du composé correspondant dans lequel $R_6$ représente un groupe cyano au moyen d'hydrure de diisobutylaluminium ;

k) lorsque $R_6$ représente un groupe $-NR_3COR_5$, la réaction du composé correspondant dans lequel $R_6$ représente un groupe $-NHR_3$ avec un composé acylique de formule $R_5CO$-X dans laquelle X représente un groupe partant ;

l) lorsque $R_6$ représente un groupe amide $-CONR_3R_4$, l'hydrolyse acide du composé correspondant dans lequel $R_6$ représente un groupe ester $-CO_2R_5$ suivi de la transformation de l'acide carboxylique ainsi obtenu en le chlorure d'acide correspondant, puis de la transformation en l'amide par traitement avec une amine de formule $H-NR_3R_4$ ;

m) lorsque $R_6$ représente un groupe $-CSNR_3R_4$, la réaction de composés dans lesquels $R_6$ représente un groupe $-CONR_3R_4$ avec le réactif de Lawesson [2,4-disulfure de [2,4bis(4-méthoxyphényl)-1,3-dithia-2,4-diphosétane] ;

n) lorsque $R_6$ représente un groupe choisi entre $R_5$, un groupe alcényle contenant jusqu'à 6 atomes de carbone pouvant facultativement être substitués par un ou plusieurs atomes d'halogènes ; ou un groupe cycloalkyle contenant 3 à 6 atomes de carbone qui peuvent être facultativement substitués par un ou plusieurs groupes $R_5$ ou par un ou plusieurs atomes d'halogènes ou un groupe $-COOR_5$ ;

par réaction d'un composé de formule générale (V) :

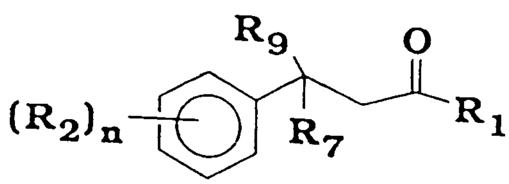

dans laquelle $R^9$ représente un groupe choisi entre $R_5$ ; un groupe alcényle contenant jusqu'à 6 atomes de carbone qui peut facultativement être substitué par un ou plusieurs atomes d'halogènes ; ou un groupe cycloalkyle contenant 3 à 6 atomes de carbone qui peut être facultativement substitué par un ou plusieurs groupes $R_5$ ou par un ou plusieurs atomes d'halogènes ou un groupe $-COOR_5$ ;

avec un orthoformiate de trialkyle en présence d'un catalyseur acide en utilisant un sel d'hydroxylamine ;

o) lorsque $R_6$ est un groupe $-CO_2R_5$, par hydrolyse du groupe cyano du composé correspondant de formule générale (I) dans laquelle $R_6$ est un groupe cyano en l'acide carboxylique correspondant qui est ensuite estérifié ;

et la transformation facultative du composé de formule (I) ainsi obtenu en un sel acceptable en agriculture de ce composé.

**5.** Composition herbicide, qui comprend comme ingrédient actif une quantité à effet herbicide d'un 4-benzylisoxazole suivant l'une quelconque des revendications 1 à 3 ou d'un sel acceptable en agriculture de ce composé, en association avec un diluant ou support et/ou un agent tensioactif acceptables en agriculture.

**6.** Composition herbicide suivant la revendication 5, qui comprend 0,05 à 90 % en poids d'ingrédient actif.

**7.** Composition herbicide suivant la revendication 5 ou 6, qui est sous la forme liquide et qui contient 0,05 à 25 % en poids d'agent tensioactif.

**8.** Compositions herbicides suivant l'une quelconque des revendications 5 à 7, sous la forme d'un concentré de suspension aqueuse, d'une poudre mouillable, d'une poudre soluble ou dispersable dans l'eau, d'un concentré liquide soluble dans l'eau, d'un concentré liquide en suspension émulsionnable, d'un produit granulaire ou d'un concentré émulsionnable.

9. Procédé pour combattre la croissance de mauvaises herbes en un lieu, qui comprend l'application à ce lieu d'une quantité à effet herbicide d'un dérivé de 4-benzylisoxazole suivant l'une quelconque des revendications 1 à 3 ou d'un sel acceptable en agriculture de ce dérivé.

10. Procédé suivant la revendication 9, dans lequel le lieu est une zone utilisée ou destinée à être utilisée pour faire croître des plantes cultivées et le composé est appliqué à un taux d'application de 0,01 kg à 4,0 kg par hectare.

11. Procédé suivant la revendication 9, dans lequel le lieu est une zone qui n'est pas une zone de culture et le composé est appliqué à un taux d'application de 1,0 kg à 20,0 kg par hectare.

12. Article de fabrication comprenant au moins un dérivé d'isoxazole suivant l'une quelconque des revendications 1 à 3 ou une composition herbicide suivant l'une quelconque des revendications 5 à 8 et de préférence un concentré herbicide qui doit être dilué avant son utilisation, comprenant au moins l'un des dérivés d'isoxazole contenu dans un récipient pour le ou les dérivés mentionnés ci-dessus ou une composition herbicide et des instructions physiquement associées au récipient mentionné ci-dessus, indiquant la manière dont le ou les dérivés ou la composition herbicide mentionnée ci-dessus, contenus dans ce récipient, doivent être utilisés pour combattre la croissance de mauvaises herbes.

**Revendications pour l'Etat contractant suivant : ES**

1. Procédé de production d'un dérivé de 4-benzylisoxazole de formule générale (I) :

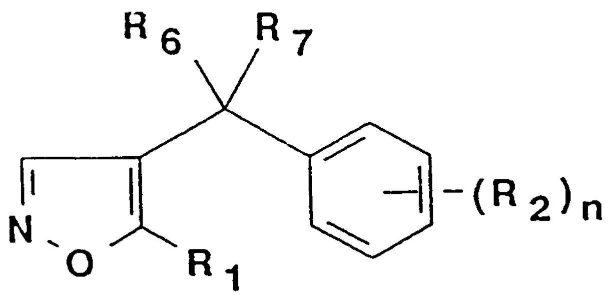

(I)

dans laquelle $R_1$ représente :

un groupe alkyle, alcényle ou alcynyle à chaîne droite ou à chaîne ramifiée contenant jusqu'à 6 atomes de carbone, qui peut facultativement être substitué par un ou plusieurs atomes d'halogènes ; ou bien

un groupe cycloalkyle contenant 3 à 6 atomes de carbone, qui peut être facultativement substitué par un ou plusieurs groupes $R_5$ et un ou plusieurs atomes d'halogènes ou un groupe $-COOR_5$ ; ou bien

un groupe cycloalcényle contenant 5 ou 6 atomes de carbone, qui peut être facultativement substitué par un ou plusieurs groupes $R_5$ ou par un ou plusieurs atomes d'halogènes ou un groupe $COOR_5$ ; ou bien

un groupe aryle ou aralkyle de formule générale $[(R_2)_q\text{phényl}]-[C(R_3)(R_4)]_p$-(le groupe aryle est généralement en $C_6$ à $C_{10}$ et le groupe aralkyle est généralement en $C_7$ à $C_{11}$) ; ou bien

un groupe ester, $COOR_5$ ; ou bien

un groupe aldéhyde ou acyle, $COR_3$ ; ou bien

un groupe cyano ou nitro ; ou bien

un groupe amino, $-NR_3R_4$ ; ou bien

un atome d'halogène ;

$R_2$ représente :

un groupe nitro ou cyano ; ou

un atome d'halogène ; ou

un groupe $R_5$ ; ou

un groupe sulfényle, sulfinyle ou sulfonyle, $-S(O)_mR_5$ ; ou

un groupe sulfamoyle, $-SO_2NR_3R_4$ ; ou

un groupe ester, $-COOR_5$ ; ou
un groupe acyle ou aldéhyde, $-COR_3$ ; ou
un groupe carbamoyle ou thiocarbamoyle $-CONR_3R_4$ ou $-CSNR_3R_4$ ; ou
un groupe alkoxy, $-OR_5$ ; ou
un groupe alkyle (ayant 1 à 3 atomes de carbone) substitué par $-OR_5$ ;
$R_3$ et $R_4$, qui peuvent être identiques ou différents, représentent chacun :
un atome d'hydrogène ou un groupe alkyle à chaîne droite ou à chaîne ramifiée contenant jusqu'à 6 atomes de carbone, qui est facultativement substitué par un ou plusieurs atomes d'halogènes ;
$R_5$ représente :
un groupe alkyle à chaîne droite ou à chaîne ramifiée contenant jusqu'à 6 atomes de carbone, qui est facultativement substitué par un ou plusieurs atomes d'halogènes ;
$R_6$ représente :
un atome d'hydrogène ; ou
un groupe hydroxy, OH ; ou
un atome d'halogène ; ou
un groupe $R_5$ ; ou
un groupe alcényle contenant jusqu'à 6 atomes de carbone, qui peut être facultativement substitué par un ou plusieurs atomes d'halogènes ; ou
un groupe cycloalkyle contenant 3 à 6 atomes de carbone qui peut être facultativement substitué par un ou plusieurs groupes $R_5$ ou par un ou plusieurs atomes d'halogènes ou un groupe $-COOR_5$ ; ou bien
un groupe sulfényle, sulfinyle ou sulfonyle, $-S(O)_mR_5$ ;
un groupe ester, $-COOR_5$ ; ou
un groupe cyano ; ou
un groupe acyle ou aldéhyde, $-COR_3$ ; ou
un groupe carbamoyle ou thiocarbamoyle, $-CONR_3R_4$ ou $-CSNR_3R_4$ ;
ou
un groupe alkoxy, $-OR_5$ ; ou
un groupe phénoxy, -O-phényl-$(R_2)_q$ ; ou
un groupe benzyloxy, $-OCH_2$-phényl-$(R_2)_q$ ; ou
un groupe acyloxy, $-OCOR_8$ ; ou
un groupe benzoyloxy, -OCO-phényl-$(R_2)_q$ ; ou
un groupe -OCO-Het1 ; ou
un groupe carbamoyloxy, $-OCONR_3R_4$ ; ou
un groupe alkylsulfonyloxy, $-OSO_2R_8$ ; ou
un groupe phénylsulfonyloxy, $-OSO_2$-phényl-$(R_2)_q$,
un groupe sulfamoyloxy, $-OSO_2NR_3R_4$ ; ou
un groupe amino, $-NR_3R_4$ ; ou
un groupe acylamino, $-NR_3COR_5$ ; ou
un groupe Het2 ;
$R_7$ représente :
un atome d'hydrogène ; ou
un groupe $R_5$ ;
ou bien $R_6$ et $R_7$ peuvent former avec l'atome de carbone auquel ils sont attachés, un cétal $((OR_5)_2)$, ou un thiocétal $((SR_5)_2)$ ou un cétal cyclique ou un thiocétal cyclique contenant 5 ou 6 atomes de carbone dans le noyau, facultativement substitué par un ou plusieurs groupes $R_5$ ;
$R_8$ représente :
un groupe alkyle ou alcényle à chaîne droite ou à chaîne ramifiée contenant jusqu'à 6 atomes de carbone, qui peut facultativement être substitué par un ou plusieurs atomes d'halogènes ; ou
un groupe cycloalkyle contenant 3 à 6 atomes de carbone qui peut facultativement être substitué par un ou plusieurs groupes $R_5$ ou par un ou plusieurs atomes d'halogènes ou un groupe $-COOR_5$ ;
Het1 représente un hétérocycle contenant 5 ou 6 atomes dans le noyau, un ou plusieurs de ces atomes consistant en un hétéroatome choisi entre azote, soufre et oxygène, les atomes de carbone du noyau étant facultativement substitués par un groupe $R_2$ ;
Het2 représente un groupe hétérocyclique choisi entre : pyrrol-1-yle, pyrazole-1-yle, imidazole-1-yle, 1,2,4-triazole-4-yle, 1,2,4-triazoyl-1-yle, 1,2,3-triazole-1-yle ou 1,2,3-triazole-2-yle ; chacun d'eux pouvant facultativement être substitué par un ou plusieurs groupes $R_2$ ;
m a la valeur zéro, 1 ou 2 ;

n représente un nombre entier de 1 à 5 ;
q est égal à zéro ou à un nombre entier de 1 à 5 ;
p a la valeur zéro ou 1 ;
ou d'un sel acceptable en agriculture de ce dérivé,

procédé qui comprend :

a) lorsque $R_6$ est un groupe OH et $R_7$ représente l'atome d'hydrogène, la réduction d'un composé de formule générale (II) :

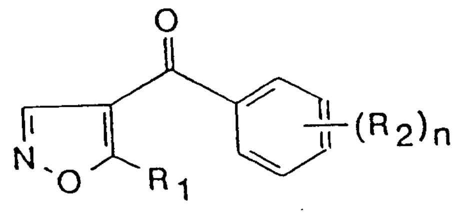

dans laquelle les autres symboles sont tels que définis ci-dessus ;

b) lorsque $R_6$ est un groupe -OH et $R_7$ est un groupe $R_5$, la réaction d'un composé de formule générale (II) avec un réactif organométallique approprié dans un solvant inerte ;

c) lorsque $R_6$ est un groupe $-OCOR_8$ ou un groupe -OCO-phényl-$(R_2)_q$ ou un groupe $-OCH_2$-phenyl-$(R_2)_q$ ou un groupe -O-phényl-$(R_2)_q$ ou un groupe -OCO-Het1 ou un groupe $-OCONR_3R_4$ ou un groupe $-OSO_2R_8$ ou un groupe $-OSO_2$-phényl-$(R_2)_q$ ou un groupe $-OSO_2NR_3R_4$ et $R_7$ est un atome d'hydrogène ou un groupe $R_5$,

la réaction d'un composé de formule générale (I) dans laquelle $R_6$ est un groupe hydroxy et $R_7$ est l'hydrogène ou un groupe $R_5$, avec le composé halogéné correspondant en présence d'une base convenable ;

d) lorsque $R_6$ est un atome de chlore ou de brome et $R_7$ est un atome d'hydrogène ou un groupe $R_5$, la réaction d'un composé de formule générale (I) dans laquelle $R_6$ est un groupe hydroxy et $R_7$ est un atome d'hydrogène ou un groupe $R_5$ avec un agent d'halogénation ;

e) lorsque $R_6$ est un atome d'halogène ou un groupe cyano et $R_7$ est un atome d'hydrogène ou un groupe $R_5$, tout d'abord la transformation du groupe hydroxyle $R_6$ d'un composé de formule générale (I) dans laquelle $R_6$ est un groupe hydroxyle et $R_7$ est un atome d'hydrogène ou un groupe $R_5$ en un groupe partant, suivie de la réaction avec un halogénure de métal alcalin, un halogénure de tétraalkylammonium ou un cyanure de métal alcalin ;

f) lorsque $R_6$ est un groupe $-OR_5$ ou un groupe $-SR_5$ ou un groupe Het2 ou un groupe $-NR_3R_4$, le remplacement de l'atome d'halogène d'un composé de formule générale (I) dans laquelle $R_6$ est un atome d'halogène par un groupe nucléophile convenable dans un solvant inerte ;

g) lorsque $R_6$ est un groupe $-SOR_5$ ou un groupe $-SO_2R_5$, l'oxydation du composé correspondant de formule générale (I) dans laquelle $R_6$ est un groupe $-SR_5$ ;

h) lorsque $R_6$ est un groupe $-OR_5$ ou un groupe $-SR_5$ et $R_7$ est un groupe $-OR_5$ ou un groupe $-SR_5$, ou bien $R_6$ et $R_7$ représentent un cétal cyclique ou un thiocétal cyclique, le traitement de composés de formule générale (II) avec l'alcool ou le thio approprié dans un solvant inerte en présence d'un catalyseur acide ;

i) lorsque $R_6$ représente un groupe $-COR_3$ dans lequel $R_3$ exclue l'hydrogène, la réaction du composé correspondant dans lequel $R_6$ représente un groupe cyano avec un réactif organométallique dans un solvant inerte ;

j) lorsque $R_6$ représente un groupe aldéhyde $-COR_3$ dans lequel $R_3$ est l'hydrogène, la réduction du composé correspondant dans lequel $R_6$ représente un groupe cyano au moyen d'hydrure de diisobutylaluminium ;

k) lorsque $R_6$ représente un groupe $-NR_3COR_5$, la réaction du composé correspondant dans lequel $R_6$ représente un groupe $-NHR_3$ avec un composé acylique de formule $R_5CO$-X dans laquelle X représente un groupe partant ;

l) lorsque $R_6$ représente un groupe amide $-CONR_3R_4$, l'hydrolyse acide du composé correspondant dans lequel $R_6$ représente un groupe ester $-CO_2R_5$ suivi de la transformation de l'acide carboxylique ainsi obtenu en le chlorure d'acide correspondant, puis de la transformation en l'amide par traitement avec une amine de formule $H-NR_3R_4$ ;

m) lorsque $R_6$ représente un groupe $-CSNR_3R_4$, la réaction de composés dans lesquels $R_6$ représente un groupe $-CONR_3R_4$ avec le réactif de Lawesson [2,4-disulfure de [2,4bis(4-méthoxyphényl)-1,3-dithia-2,4-diphosétane] ;

n) lorsque $R_6$ représente un groupe choisi entre $R_5$, un groupe alcényle contenant jusqu'à 6 atomes de carbone pouvant facultativement être substitués par un ou plusieurs atomes d'halogènes ; ou un groupe cycloalkyle contenant 3 à 6 atomes de carbone qui peuvent être facultativement substitués par un ou plusieurs groupes $R_5$ ou par un ou plusieurs atomes d'halogènes ou un groupe $-COOR_5$ ;

par réaction d'un composé de formule générale (V) :

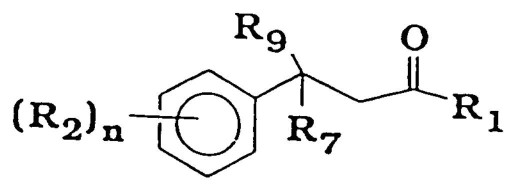

dans laquelle $R^9$ représente un groupe choisi entre $R_5$ ; un groupe alcényle contenant jusqu'à 6 atomes de carbone qui peut facultativement être substitué par un ou plusieurs atomes d'halogènes ; ou un groupe cycloalkyle contenant 3 à 6 atomes de carbone qui peut être facultativement substitué par un ou plusieurs groupes $R_5$ ou par un ou plusieurs atomes d'halogènes ou un groupe $-COOR_5$ ;

avec un orthoformiate de trialkyle en présence d'un catalyseur acide en utilisant un sel d'hydroxylamine ;

o) lorsque $R_6$ est un groupe $-CO_2R_5$, par hydrolyse du groupe cyano du composé correspondant de formule générale (I) dans laquelle $R_6$ est un groupe cyano en l'acide carboxylique correspondant qui est ensuite estérifié ;

et la transformation facultative du composé de formule (I) ainsi obtenu en un sel acceptable en agriculture de ce composé.

**2.** Procédé suivant la revendication 1, pour la préparation d'un composé de formule générale (I) dans laquelle :

a) l'un des groupes représentés par $(R_2)_n$ est en position ortho sur le noyau phényle ; et/ou

b) lorsqu'il y a deux groupes représentés par $(R_2)_n$, ils sont en positions 2 et 4 sur le noyau phényle ; et/ou

c) $R_1$ est un groupe alkyle facultativement substitué par un halogène ; ou un groupe cycloalkyle facultativement substitué par un radical alkyle ou un halogène ; et/ou

d) $R_2$ est un halogène ; ou un groupe nitro ; ou un groupe $R_5$ ; ou un groupe $-S(O)_mR_5$ ; ou $-OR_5$ ; ou un groupe alkyle substitué par $-OR_5$ ; ou un groupe $-COOR_5$ ; et/ou

e) $R_5$ est un groupe alkyle (ayant 1 à 3 atomes de carbone) facultativement substitué par du fluor ou du chlore ; et/ou

f) $R_6$ est de l'hydrogène, un halogène, un groupe -OH, $-OR_5$, $-OCOR_8$ ou un groupe alkyle (ayant un à quatre atomes de carbone) facultativement substitués par un ou plusieurs atomes d'halogènes ; et/ou

g) l'un des groupes $R_6$ et $R_7$ est un atome d'hydrogène ; et/ou

h) $R_7$ est un atome d'hydrogène ou un groupe alkyle contenant un à quatre atomes de carbone ; et/ou

i) $R_8$ est un groupe alkyle contenant un à quatre atomes de carbone facultativement substitués par un ou plusieurs atomes d'halogènes ; et/ou

j) n a la valeur 2 ou 3.

**3.** Procédé suivant la revendication 1 ou 2 pour la préparation d'un composé de formule générale (I) qui est :

le 4-[hydroxy-(2-nitro-4-trifluorométhylphényl)méthyl]-5-cyclopropylisoxazole ;
le 4-[hydroxy-(2-nitro-4-trifluorométhylphényl)méthyl]-5-méthylisoxazole ;
le 4-[hydroxy-(2-chloro-3-éthoxy-4-méthanesulfonylphényl)méthyl]-5-cyclopropylisoxazole ;
le 4-[acétoxy-(2-nitro-4-trifluorométhylphényl)méthyl]-5-cyclopropylisoxazole ;
le 4-[chloro-(2-nitro-4-trifluorométhylphényl)méthyl]-5-cyclopropylisoxazole ;

le 4-[bromo-(2-nitro-4-trifluorométhylphényl)méthyl]-5-cyclopropylisoxazole ;
le 4-[N,N-diméthylamino-(2-nitro-4-trifluorométhylphényl)méthyl]-5-cyclopropylisoxazole ;
le 4-[hydroxy-(2-chloro-4-méthanesulfonylphényl)méthyl]-5-cyclopropylisoxazole ;
le4-[méthoxy-(2-nitro-4-trifluorométhylphényl)méthyl]-5-cyclopropylisoxazole ;
le4-[hydroxy-(2-nitro-4-méthylphényl)méthyl]-5-cyclopropylisoxazole ;
le 4-[hydroxy-(2-chloro-3-éthoxy-4-éthanesulfonylphényl)méthyl]-5-méthylisoxazole ;
le 4-[hydroxy-(2-chloro-3-éthoxy-4-éthanesulfonylphényl)méthyl]-5-cyclopropylisoxazole ;
le 4-[acétoxy-(2-chloro-4-méthanesulfonylphényl)méthyl]-5-cyclopropylisoxazole ;
le 4-[méthoxy-(2-chloro-4-méthanesulfonylphényl)méthyl]-5-cyclopropylisoxazole ;
le 4-[hydroxy-(2-chloro-4-fluorophényl)méthyl]-5-cyclopropylisoxazole ;
le 4-[hydroxy-(2-trifluorométhyl-4-méthanesulfonylphényl)méthyl]-5-cyclopropylisoxazole ;
le 4-[acétoxy-(2-trifluorométhyl-4-méthanesulfonylphényl)méthyl]-5-cyclopropylisoxazole ;
le 4-[méthoxy-(2-trifluorométhyl-4-méthanesulfonylphényl)méthyl]-5-cyclopropylisoxazole ;
le 4-[bromo-(2-trifluorométhyl-4-méthanesulfonylphényl)méthyl]-5-cyclopropylisoxazole ;
le 4-[chloro-(2-trifluorométhyl-4-méthanesulfonylphényl)méthyl]-5-cyclopropylisoxazole ;
le 4-[trifluoroacétoxy-(2-trifluorométhyl-4-méthanesulfonylphényl)-méthyl]-5-cyclopropylisoxazole ;
le 4-[hydroxy-(2-méthanesulfonylphényl)méthyl]-5-cyclopropylisoxazole ;
le 4-[hydroxy(2,3-dichloro-4-méthanesulfonylphényl)méthyl]-5-(1-méthylcyclopropyl)isoxazole ;
le 4-[hydroxy-(2-méthanesulfonyl-4-chlorophényl)méthyl]-5-cyclopropylisoxazole ;
le4-[hydroxy-(2-bromo-4-méthanesulfonylphényl)méthyl]-5-cyclopropylisoxazole ;
le 4-[hydroxy-(2-méthanesulfonyl-4-bromophényl)méthyl]-5-cyclopropylisoxazole ;
le 4-[hydroxy-(2-méthyl-4-méthanesulfonylphényl)méthyl]-5-cyclopropylisoxazole ;
le 4-[hydroxy-(2-chloro-4-méthanethiophényl)méthyl]-5-cyclopropylisoxazole ;
le 4-[hydroxy-(2-méthanesulfonyl-4-trifluorométhylphényl)méthyl]-5-cyclopropylisoxazole ;
le 4-[hydroxy-(2-chloro-4-méthanesulfinylphényl)méthyl]-5-cyclopropylisoxazole ;
le 4-[hydroxy-(2-trifluorométhyl-4-méthanethiophényl)méthyl]-5-cyclopropylisoxazole ;
le 4-[hydroxy-(2-fluoro-4-méthanesulfonylphényl)méthyl]-5-cyclopropylisoxazole ;
le 4-[hydroxy-(2-méthanesulfonyl-4-chlorophényl)méthyl]-5-isopropylisoxazole ; ou
le 4-[hydroxy-(2-méthanesulfonyl-4-chlorophényl)méthyl]-5-(1-méthylcyclopropyl)isoxazole.

**4.** Procédé de production d'une composition herbicide, qui comprend la formulation, comme ingrédient actif, d'une quantité à effet herbicide d'un 4-benzylisoxazole, telle que définie dans l'une quelconque des revendications 1 à 3 ou un sel acceptable en agriculture de ce composé, avec un diluant ou support et/ou un agent tensioactif acceptable en agriculture.

**5.** Procédé suivant la revendication 4, dans lequel la composition herbicide comprend 0,05 à 90 % en poids d'ingrédient actif.

**6.** Procédé suivant la revendication 4 ou 5, dans lequel la composition herbicide est sous la forme liquide et contient 0,05 à 25 % d'agent tensioactif.

**7.** Procédé suivant l'une quelconque des revendications 4 à 6, dans lequel la composition herbicide est sous la forme d'un concentre de suspension aqueuse, d'une poudre mouillable, d'une poudre soluble ou dispersable dans l'eau, d'un concentré liquide soluble dans l'eau, d'un concentré liquide en suspension émulsionnable, d'une composition granulaire ou d'un concentré émulsionnable.

**8.** Procédé pour combattre la croissance de mauvaises herbes en un lieu, qui comprend l'application à ce lieu d'une quantité à effet herbicide d'un dérivé de 4-benzylisoxazole suivant l'une quelconque des revendications 1 à 3 ou d'un sel acceptable en agriculture de ce dérivé.

**9.** Procédé suivant la revendication 8, dans lequel le lieu est une zone utilisée ou destinée à être utilisée pour faire croître des plantes cultivées et le composé est appliqué à un taux d'application de 0,01 kg à 4,0 kg par hectare.

**10.** Procédé suivant la revendication 8, dans lequel le lieu est une zone qui n'est pas une zone de culture et le composé est appliqué à un taux d'application de 1,0 kg à 20,0 kg par hectare.

**11.** Procédé de production d'un article de fabrication comprenant au moins un dérivé d'isoxazole tel que défini dans l'une quelconque des revendications 1 à 3 ou une composition herbicide telle que définie dans l'une quelconque des revendications 4 à 7 et, de préférence, un concentré herbicide qui doit être dilué avant son utilisation, procédé selon lequel le ou les dérivés d'isoxazole ou la composition herbicide sont contenus dans un récipient et des instructions sont mises en association physique avec ce récipient, indiquant la manière dont le ou les dérivés ou la composition herbicide mentionnés ci-dessus contenus dans ce récipient, doivent être utilisés pour combattre la croissance de mauvaises herbes.